# EUROPEAN PATENT APPLICATION

(11) **EP 3 159 007 A1**
(43) Date of publication of application: **26.04.2017**
(21) Application number: 16194112.5
(22) Date of filing: 17.10.2016
(51) Int. Cl.: A61K 39/395, C07K 16/24, A61K 39/00

(54) **PHARMACEUTICAL COMPOSITION FOR TREATING RHEUMATOID ARTHRITIS**

(30) Priority: 19.10.2015 US 201562243222 P
(71) Applicant: Eisai R&D Management Co., Ltd., Tokyo 112-8088 (JP)
(72) Inventor: Yasuda, Nobuyuki, Tsukuba-shi Ibaraki 300-2635 (JP); Tago, Fumitoshi, Tokyo 112-8088 (JP); Baba, Hiroko, Tokyo 112-8088 (JP); Nakano, Tomohisa, Tokyo 112-8088 (JP); Mori, Masahiko, Tokyo 112-8088 (JP)
(74) Representative: Hoffmann Eitle

(57) **Abstract**

An object of the present invention is to provide a pharmaceutical composition comprising an anti-fractalkine antibody, wherein the pharmaceutical composition brings about therapeutically effective amelioration in rheumatoid arthritis after administration to a human subject. The present invention provides a pharmaceutical composition for the treatment of rheumatoid arthritis. The pharmaceutical composition comprises an anti-fractalkine antibody and a pharmaceutically acceptable excipient and is used such that at least 100 mg per dose of the anti-fractalkine antibody is subcutaneously administered to a human in need thereof.

SEQUENCE LISTING

<110>  EISAI R&D MANAGEMENT CO., LTD.

<120>  A pharmaceutical composition for treating rheumatoid arthritis

<130>  ESAP1506-EP

<160>  26

<170>  PatentIn version 3.5

<210>  1
<211>  119
<212>  PRT
<213>  Artificial Sequence

<220>
<223>  Synthetic polypeptide

<400>  1

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Glu Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser
        115

<210>  2
<211>  107
<212>  PRT
<213>  Artificial Sequence

<220>
<223>  Synthetic polypeptide

<400>  2

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105


<210>  3
<211>  19
<212>  PRT
<213>  Artificial Sequence

<220>
<223>  Synthetic polypeptide

<400>  3

Met Glu Trp Ser Trp Val Phe Leu Phe Phe Leu Ser Val Thr Thr Gly
1               5                   10                  15

Val His Ser


<210>  4
<211>  326
<212>  PRT
<213>  Artificial Sequence

<220>
<223>  Synthetic polypeptide

<400>  4

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Ala Ala Ala Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys

275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  5
<211>  464
<212>  PRT
<213>  Artificial Sequence

<220>
<223>  Synthetic polypeptide

<400>  5

Met Glu Trp Ser Trp Val Phe Leu Phe Phe Leu Ser Val Thr Thr Gly
1               5                   10                  15

Val His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Asn Tyr Tyr Ile His Trp Val Lys Gln Ala Pro Gly Gln Gly Leu
    50                  55                  60

Glu Trp Ile Gly Trp Ile Tyr Pro Gly Asp Gly Ser Pro Lys Phe Asn
65                  70                  75                  80

Glu Arg Phe Lys Gly Arg Thr Thr Leu Thr Ala Asp Lys Ser Thr Asn
                85                  90                  95

Thr Ala Tyr Met Leu Leu Ser Ser Leu Arg Ser Glu Asp Thr Ala Val
            100                 105                 110

Tyr Phe Cys Ala Thr Gly Pro Thr Asp Gly Asp Tyr Phe Asp Tyr Trp
        115                 120                 125

Gly Gln Gly Thr Thr Val Thr Val Ser Ser Ala Ser Thr Lys Gly Pro
    130                 135                 140

Ser Val Phe Pro Leu Ala Pro Cys Ser Arg Ser Thr Ser Glu Ser Thr
145                 150                 155                 160

Ala Ala Leu Gly Cys Leu Val Lys Asp Tyr Phe Pro Glu Pro Val Thr
                165                 170                 175

Val Ser Trp Asn Ser Gly Ala Leu Thr Ser Gly Val His Thr Phe Pro
            180                 185                 190

Ala Val Leu Gln Ser Ser Gly Leu Tyr Ser Leu Ser Ser Val Val Thr
        195                 200                 205

Val Pro Ser Ser Asn Phe Gly Thr Gln Thr Tyr Thr Cys Asn Val Asp
    210                 215                 220

His Lys Pro Ser Asn Thr Lys Val Asp Lys Thr Val Glu Arg Lys Cys
225                 230                 235                 240

Cys Val Glu Cys Pro Pro Cys Pro Ala Pro Pro Ala Ala Ala Pro Ser
                245                 250                 255

Val Phe Leu Phe Pro Pro Lys Pro Lys Asp Thr Leu Met Ile Ser Arg
            260                 265                 270

Thr Pro Glu Val Thr Cys Val Val Val Asp Val Ser His Glu Asp Pro
        275                 280                 285

Glu Val Gln Phe Asn Trp Tyr Val Asp Gly Val Glu Val His Asn Ala
    290                 295                 300

Lys Thr Lys Pro Arg Glu Glu Gln Phe Asn Ser Thr Phe Arg Val Val
305                 310                 315                 320

Ser Val Leu Thr Val Val His Gln Asp Trp Leu Asn Gly Lys Glu Tyr
                325                 330                 335

Lys Cys Lys Val Ser Asn Lys Gly Leu Pro Ala Pro Ile Glu Lys Thr
            340                 345                 350

Ile Ser Lys Thr Lys Gly Gln Pro Arg Glu Pro Gln Val Tyr Thr Leu
        355                 360                 365

Pro Pro Ser Arg Glu Glu Met Thr Lys Asn Gln Val Ser Leu Thr Cys
    370                 375                 380

Leu Val Lys Gly Phe Tyr Pro Ser Asp Ile Ala Val Glu Trp Glu Ser
385                 390                 395                 400

Asn Gly Gln Pro Glu Asn Asn Tyr Lys Thr Thr Pro Pro Met Leu Asp

405                 410                 415

Ser Asp Gly Ser Phe Phe Leu Tyr Ser Lys Leu Thr Val Asp Lys Ser
            420                 425                 430

Arg Trp Gln Gln Gly Asn Val Phe Ser Cys Ser Val Met His Glu Ala
        435                 440                 445

Leu His Asn His Tyr Thr Gln Lys Ser Leu Ser Leu Ser Pro Gly Lys
    450                 455                 460

<210>  6
<211>  20
<212>  PRT
<213>  Artificial Sequence

<220>
<223>  Synthetic polypeptide

<400>  6

Met Ser Val Pro Thr Gln Val Leu Gly Leu Leu Leu Leu Trp Leu Thr
1               5                   10                  15

Asp Ala Arg Cys
            20

<210>  7
<211>  107
<212>  PRT
<213>  Artificial Sequence

<220>
<223>  Synthetic polypeptide

<400>  7

<210>  8
<211>  234
<212>  PRT
<213>  Artificial Sequence

<220>
<223>  Synthetic polypeptide

<400>  8

Met Ser Val Pro Thr Gln Val Leu Gly Leu Leu Leu Leu Trp Leu Thr
1               5                   10                  15

Asp Ala Arg Cys Asp Ile Gln Met Thr Gln Ser Pro Ser Ser Leu Ser
            20                  25                  30

Ala Ser Val Gly Asp Arg Val Thr Ile Thr Cys Arg Ala Ser Gly Asn
        35                  40                  45

Ile His Asn Phe Leu Ala Trp Tyr Gln Gln Lys Pro Gly Lys Ala Pro
    50                  55                  60

Lys Leu Leu Ile Tyr Asn Glu Lys Thr Leu Ala Asp Gly Val Pro Ser
65                  70                  75                  80

Arg Phe Ser Gly Ser Gly Ser Gly Thr Asp Tyr Thr Leu Thr Ile Ser
                85                  90                  95

Ser Leu Gln Pro Glu Asp Phe Ala Thr Tyr Phe Cys Gln Gln Phe Trp
            100                 105                 110

Ser Thr Pro Tyr Thr Phe Gly Gly Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr

180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  9
<211>  1417
<212>  DNA
<213>  Artificial Sequence

<220>
<223>  Synthetic oligonucleotide

<400>  9
aagcttgccg ccaccatgga atggtcctgg gtgttcctgt tcttcctgtc cgtgaccacc      60

ggcgtgcact cccaggtgca gctggtgcag tctggcgccg aagtgaagaa acctggcgcc     120

tccgtgaagg tgtcctgcaa ggcctccggc tacaccttca ccaactacta catccactgg     180

gtgaaacagg ccccaggaca gggcctggaa tggatcggct ggatctaccc cggcgacggc     240

tcccccaagt tcaacgagcg gttcaagggc cggaccaccc tgaccgccga caagtccacc     300

aacaccgcct acatgctgct gtcctccctg cggagcgagg ataccgccgt gtacttctgc     360

gccaccggcc ctaccgacgg cgactacttc gactactggg gccagggcac caccgtgacc     420

gtgtcctctg cctccaccaa gggcccctcc gtgttccctc tggccccttg ctcccggtcc     480

acctctgagt ctaccgccgc tctgggctgc ctggtcaaag actacttccc cgagcctgtg     540

acagtgtcct ggaactctgg cgccctgacc tctggagtgc ataccttccc tgccgtgctg     600

cagtcatccg gcctgtactc cctgtcctcc gtggtgacag tgccctcctc caacttcggc     660

acccagacct acacctgtaa cgtggaccac aagccctcca acaccaaggt ggacaagacc     720

gtggaacgga agtgctgcgt ggaatgcccc ccctgtcctg cccctcctgc cgccgctcct     780

tccgtgtttc tgttcccccc aaagcccaag gacaccctga tgatctcccg gacccccgaa     840

gtgacctgcg tggtggtgga cgtgtcccac gaggaccccg aggtgcagtt caattggtac     900

gtggacggcg tggaagtgca caacgccaag accaagccca gagaggaaca gttcaactcc     960

accttccggg tggtgtccgt gctgaccgtg gtgcaccagg actggctgaa cggcaaagag    1020

tacaagtgca aggtctccaa caagggcctg cctgccccca tcgaaaagac catcagcaag    1080

accaagggcc agccccgcga gccccaggtg tacacactgc cccccagccg ggaagagatg    1140

accaagaacc aggtgtccct gacctgtctg gtgaaaggct tctacccctc cgatatcgcc    1200

gtggaatggg agtccaacgg acagcccgag aacaactaca agaccacccc ccccatgctg 1260

gactccgacg gctcattctt cctgtactcc aagctgacag tggacaagtc ccggtggcag 1320

cagggcaacg tgttctcctg cagcgtgatg cacgaggccc tgcacaacca ctacacccag 1380

aagtccctga gcctgagccc cggcaagtga tgaattc 1417

<210> 10
<211> 727
<212> DNA
<213> Artificial Sequence

<220>
<223> Synthetic oligonucleotide

<400> 10
aagcttgccg ccaccatgtc cgtgcccacc caggtgctgg gcctgctgct gctgtggctg 60

accgacgcca gatgcgacat ccagatgacc cagtccccct ccagcctgtc cgcctctgtg 120

ggcgacagag tgaccatcac ctgtcgggcc tccggcaaca tccacaactt tctggcctgg 180

tatcagcaga agcccggcaa ggcccccaag ctgctgatct acaacgaaaa gaccctggcc 240

gacggcgtgc cctccagatt ctccggctct ggctccggca ccgactacac cctgaccatc 300

tccagcctgc agcccgagga cttcgccacc tacttttgcc agcagttctg gtccaccccc 360

tacaccttcg gcggaggcac caaggtggaa atcaagcgga ccgtggccgc tccctccgtg 420

ttcatcttcc caccctccga cgagcagctg aagtccggca ccgcctccgt ggtgtgcctg 480

ctgaacaact tctacccccg cgaggccaag gtgcagtgga aggtggacaa cgccctgcag 540

tccggcaact cccaggaatc cgtcaccgag caggactcca aggacagcac ctactccctg 600

tcctccaccc tgaccctgtc caaggccgac tacgagaagc acaaggtgta cgcctgcgaa 660

gtgacccacc agggcctgtc cagccccgtg accaagtcct tcaaccgggg cgagtgctga 720

tgaattc 727

<210> 11
<211> 445
<212> PRT
<213> Artificial Sequence

<220>
<223> Synthetic polypeptide

<400> 11

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Glu Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Cys Cys Val Glu
    210                 215                 220

Cys Pro Pro Cys Pro Ala Pro Pro Ala Ala Ala Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210>  12
<211>  214
<212>  PRT
<213>  Artificial Sequence

<220>
<223>  Synthetic polypeptide

<400>  12

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  13
<211>  119
<212>  PRT
<213>  Artificial Sequence

<220>
<223>  Synthetic polypeptide

<400>  13

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Glu Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser
        115

<210>  14
<211>  107
<212>  PRT
<213>  Artificial Sequence

<220>
<223>  Synthetic polypeptide

<400>  14

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105

<210>  15

<211>  5
<212>  PRT
<213>  Artificial Sequence

<220>
<223>  Synthetic polypeptide

<400>  15

Asn Tyr Tyr Ile His
1               5


<210>  16
<211>  17
<212>  PRT
<213>  Artificial Sequence

<220>
<223>  Synthetic polypeptide

<400>  16

Trp Ile Tyr Pro Gly Asp Gly Ser Pro Lys Phe Asn Glu Arg Phe Lys
1               5                   10                  15


Gly




<210>  17
<211>  10
<212>  PRT
<213>  Artificial Sequence

<220>
<223>  Synthetic polypeptide

<400>  17

Gly Pro Thr Asp Gly Asp Tyr Phe Asp Tyr
1               5                   10


<210>  18
<211>  11
<212>  PRT
<213>  Artificial Sequence

<220>
<223>  Synthetic polypeptide

<400>  18

Arg Ala Ser Gly Asn Ile His Asn Phe Leu Ala
1               5                   10


<210>  19
<211>  7
<212>  PRT
<213>  Artificial Sequence

<220>
<223>  Synthetic polypeptide

<400>  19

Asn Glu Lys Thr Leu Ala Asp
1               5


<210>  20
<211>  9
<212>  PRT
<213>  Artificial Sequence

<220>
<223>  Synthetic polypeptide

<400>  20

Gln Gln Phe Trp Ser Thr Pro Tyr Thr
1               5


<210>  21
<211>  119
<212>  PRT
<213>  Artificial Sequence

<220>
<223>  Synthetic polypeptide

<400>  21

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Asp Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser

        115

<210>  22
<211>  119
<212>  PRT
<213>  Artificial Sequence

<220>
<223>  Synthetic polypeptide

<400>  22

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Arg Asp Lys Ser Thr Asn Thr Ala Tyr
65                  70                  75                  80

Met Glu Leu Ser Ser Leu Arg Ser Asp Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser
        115

<210>  23
<211>  119
<212>  PRT
<213>  Artificial Sequence

<220>
<223>  Synthetic polypeptide

<400>  23

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Met Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Gly Pro Thr Asp Gly Asp Tyr Phe Asp Tyr Trp Gly Gln Gly
            100                 105                 110

Thr Thr Val Thr Val Ser Ser
        115

<210>  24
<211>  119
<212>  PRT
<213>  Artificial Sequence

<220>
<223>  Synthetic polypeptide

<400>  24

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Gly Pro Thr Asp Gly Asp Tyr Phe Asp Tyr Trp Gly Gln Gly
            100                 105                 110

Thr Thr Val Thr Val Ser Ser

        115

<210>  25
<211>  107
<212>  PRT
<213>  Artificial Sequence

<220>
<223>  Synthetic polypeptide

<400>  25

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Phe Leu Val
        35                  40                  45

Tyr Asn Glu Lys Thr Leu Ala Asp Gly Val Pro Ser Arg Phe Ser Gly
    50                  55                  60

Ser Gly Ser Gly Thr Gln Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105


<210>  26
<211>  107
<212>  PRT
<213>  Artificial Sequence

<220>
<223>  Synthetic polypeptide

<400>  26

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Gln Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105

## Description

### BACKGROUND OF THE INVENTION

### Field of the Invention

The present invention relates to a pharmaceutical composition for the treatment of rheumatoid arthritis, comprising an anti-fractalkine antibody. The present application claims the priority based on U.S. Provisional Application No. 62/243,222 filed on October 19, 2015, the contents of which are incorporated herein.

### Description of the Related Art

Fractalkine (also referred to as "FKN") is a membrane-bound chemokine that is expressed on the surface of vascular endothelial cells by the inflammatory stimulus of LPS, TNF-α, IL-1, or the like. Cells expressing an FKN receptor CX3CR1 bind to membrane-bound FKN without any involvement of selectin or integrin and cause strong cell adhesion. Secreted FKN shed from the membrane-bound FKN exhibits cell-migrating activity against NK cells, T cells, and monocytes having CX3CR1.

The expression of FKN is induced on the surface of vascular endothelial cells by proinflammatory cytokines. The increased expression of FKN and the accumulation of CX3CR1⁺ cytotoxic effector lymphocytes and macrophages have been reported as to patients having rheumatoid arthritis (also referred to as "RA").

Therapeutic effects by the inhibition of FKN have previously been reported as to collagen-induced arthritis (CIA) murine models, which are known as chronic rheumatoid arthritis models (J Immunol. 2004; 173: 7010-7016). The results obtained in CIA indicate significant reduction in the clinical score of arthritis, significant decrease in the incidence of arthritis, and significant decrease in inflammatory cells in synovial membranes and bony erosion, due to anti-fractalkine antibodies. Furthermore, it is suggested that an anti-fractalkine antibody inhibiting the interaction between FKN and CX3CR1 is capable of treating inflammatory diseases including rheumatoid arthritis (WO2006/046739). Thus, as for the treatment of rheumatoid arthritis, it is suggested that an antibody that can bind to FKN and inhibit this FKN is effective for the treatment of rheumatoid arthritis.

The applicant of this application has previously disclosed a plurality of mouse anti-human fractalkine (hFKN) monoclonal antibodies (clones 1F3-1, 3A5-2, 1F3, 1G1, 2B2, 3D5, 3H7, 6D1, 7F6, and 5H7-6). Particularly, the clone 3A5-2 has been humanized because of its high neutralizing activity, binding affinity, and interspecific cross reactivity against hFKN, and designated as H3-2L4 (WO2011/052799, which is incorporated herein by reference in its entirety).

An object of the present invention is to provide a pharmaceutical composition comprising an anti-fractalkine antibody (in the present specification, also referred to as an "anti-FKN antibody"), wherein the pharmaceutical composition brings about therapeutically effective amelioration in rheumatoid arthritis after administration to a human subject.

Another object of the present invention is to provide a pharmaceutical composition comprising an anti-FKN antibody, wherein the pharmaceutical composition is used so as to bring about therapeutically effective amelioration in rheumatoid arthritis.

### SUMMARY OF THE INVENTION

The present invention encompasses the following embodiments:
[1] A pharmaceutical composition for the treatment of rheumatoid arthritis,
   the pharmaceutical composition comprising an anti-fractalkine antibody and a pharmaceutically acceptable excipient, wherein
   the anti-fractalkine antibody is an antibody wherein:
   a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
   a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
   the antibody comprises a constant region of human IgG2 isotype; and
   a Fc region of the constant region of human IgG2 isotype contains V234A and G237A mutations, and
   the pharmaceutical composition is used such that at least 100 mg per dose of the anti-fractalkine antibody is subcutaneously administered to a human.
[2] The pharmaceutical composition according to [1], wherein
   the pharmaceutical composition is used such that 100 mg to 400 mg per dose of the anti-fractalkine antibody is subcutaneously administered to a human.
[3] The pharmaceutical composition according to [1], wherein
   the pharmaceutical composition is used such that 100 mg, 200 mg, or 400 mg per dose of the anti-fractalkine antibody is subcutaneously administered to a human.
[4] The pharmaceutical composition according to [1], wherein
   when 100 mg of the anti-fractalkine antibody is subcutaneously administered by a single administration to a human,
   mean Cₘₐₓ is a value that falls within the numerical range of 80% to 125% of 10 µg/mL,
   mean AUC₍₀₋₃₃₆ₕ₎ is a value that falls within the numerical range of 80% to 125% of 2.4 × 10³ µg·h/mL, or
   mean AUC₍₀₋ₜ₎ is a value that falls within the numerical range of 80% to 125% of 3.0 × 10³ µg·h/mL.
[5] The pharmaceutical composition according to [1], wherein
   when 200 mg of the anti-fractalkine antibody is subcutaneously administered by a single administration to a human,
   mean Cₘₐₓ is a value that falls within the numerical range of 80% to 125% of 27 µg/mL,
   mean AUC₍₀₋₃₃₆ₕ) is a value that falls within the numerical range of 80% to 125% of 7.4 × 10³ µg·h/mL, or
   mean AUC₍₀₋ₜ₎ is a value that falls within the numerical range of 80% to 125% of 1.2 × 10⁴ µg·h/mL.
[6] The pharmaceutical composition according to [1], wherein
   when 400 mg of the anti-fractalkine antibody is subcutaneously administered by a single administration to a human,
   mean Cₘₐₓ is a value that falls within the numerical range of 80% to 125% of 43 µg/mL,
   mean AUC₍₀₋₃₃₆ₕ₎ is a value that falls within the numerical range of 80% to 125% of 1.2 × 10⁴ µg·h/mL, or
   mean AUC₍₀₋ₜ₎ is a value that falls within the numerical range of 80% to 125% of 2.7 × 10⁴ µg·h/mL.
[7] The pharmaceutical composition according to [1], wherein
   the pharmaceutical composition is subcutaneously administered by multiple administrations at dosing intervals of once a week to once every two weeks.
[8] The pharmaceutical composition according to any one of [1] to [7], wherein
   the pharmaceutical composition is subcutaneously administered such that the mean trough concentration of the anti-fractalkine antibody is 10 µg/mL or higher.
[9] The pharmaceutical composition according to any one of [1] to [7], wherein
   the pharmaceutical composition is subcutaneously administered such that the mean trough concentration of the anti-fractalkine antibody is 20 µg/mL or higher.
[10] A therapeutic agent for rheumatoid arthritis, comprising an anti-fractalkine antibody, wherein
   the therapeutic agent for rheumatoid arthritis is used such that 100 mg to 400 mg of the anti-fractalkine antibody is subcutaneously administered at dosing intervals of once a week to once every two weeks to a human, and
   the anti-fractalkine antibody is an antibody wherein:
   a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) ;
   a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
   the antibody comprises a constant region of human IgG2 isotype; and
   a Fc region of the constant region of human IgG2 isotype contains V234A and G237A mutations.

The present invention provides a pharmaceutical composition comprising an anti-FKN antibody, wherein the pharmaceutical composition brings about therapeutically effective amelioration in rheumatoid arthritis after administration to a human subject. The pharmaceutical composition of the present invention can be used so as to bring about a pharmacokinetic parameter useful for exerting therapeutic effects on rheumatoid arthritis.

### BRIEF DESCRIPTION OF THE DRAWINGS

FIG. 1 shows, in a time-dependent manner, the summary statistic of serum H3-2L4 concentration after a single subcutaneous administration (each dose of 50 mg, 100 mg, 200 mg, and 400 mg) of antibody H3-2L4;
FIG. 2 shows time-dependent change in the summary statistics of serum H3-2L4 concentration and serum total FKN concentration after multiple subcutaneous administrations (100 mg group) of antibody H3-2L4;
FIG. 3 shows time-dependent change in the summary statistics of serum H3-2L4 concentration and serum total FKN concentration after multiple subcutaneous administrations (200 mg group) of antibody H3-2L4;
FIG. 4 shows ACR20, ACR50, and ACR70 at week 12 of multiple subcutaneous administrations (100 mg group, 200 mg group, and 400 mg group) of antibody H3-2L4;
FIG. 5 shows change in ACR70 over 12 weeks of multiple subcutaneous administrations (100 mg group, 200 mg group, and 400 mg group) of antibody H3-2L4;
FIG. 6 shows change in DAS28-CRP over 12 weeks of multiple subcutaneous administrations (100 mg group, 200 mg group, and 400 mg group) of antibody H3-2L4; and
FIG. 7 shows change in the proportions of disease activity scores of DAS28-CRP in multiple subcutaneous administrations (100 mg group, 200 mg group, and 400 mg) of antibody H3-2L4 before administration) and at week 12 after the start of administration.

### DETAILED DESCRIPTION OF THE PREFERRED EMBODIMENTS

### 1. Summary of invention and definition

The present invention relates to a pharmaceutical composition that brings about therapeutically effective amelioration in rheumatoid arthritis.

In one embodiment of the present invention, the "therapeutically effective amelioration in rheumatoid arthritis" can mean that the therapeutically effective amelioration is exhibited for one or more of items or parameters specified in evaluation criteria for rheumatoid arthritis established in the art. Examples of such items or parameters can include, but are not limited to, ACR (American College of Rheumatology) 20 response rates, ACR50 response rates, ACR70 response rates, erythrocyte sedimentation rates (ESR), high sensitive C-reactive protein (hs-CRP), health assessment questionnaire (HAQ), simple disease activity index (SDAI), clinical disease activity index (CDAI; e.g., disease activity score using CRP (DAS28-CRP)), and Boolean remission rates.

In one embodiment of the present invention, the therapeutically effective amelioration in rheumatoid arthritis can mean at least 10%, at least 20%, at least 30%, at least 40%, at least 50%, at least 60%, at least 70%, at least 80%, or at least 90% decrease in ESR from the baseline.

In one embodiment of the present invention, the therapeutically effective amelioration in rheumatoid arthritis can mean at least 10%, at least 20%, at least 30%, at least 40%, at least 50%, at least 60%, at least 70%, at least 80%, or at least 90% decrease in hs-CRP from the baseline.

In one embodiment of the present invention, the therapeutically effective amelioration in rheumatoid arthritis can mean that the DAS28 score itself or severity evaluated on the basis of DAS28 is ameliorated. For example, the therapeutically effective amelioration in rheumatoid arthritis can mean that, in severity evaluation based on DAS28-CRP, a severe case is ameliorated to a moderate case, a moderate case is ameliorated to a mild case, or a mild case is ameliorated to remission.

In one aspect, the present invention relates to a pharmaceutical composition for the treatment of rheumatoid arthritis which brings about a therapeutically effective serum anti-FKN antibody concentration after a single administration or multiple administrations to a human subject.

In one embodiment of the present invention, the "therapeutically effective serum anti-FKN antibody concentration" is a serum concentration of 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher of the anti-FKN antibody. In an alternative embodiment of the present invention, the "therapeutically effective serum anti-FKN antibody concentration" can be a mean trough concentration of 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher of the anti-FKN antibody. In a preferred embodiment, the "therapeutically effective serum anti-FKN antibody concentration" is a mean trough concentration of 10 µg/mL or higher or 20 µg/mL or higher of the anti-FKN antibody.

In a preferred embodiment of the present invention, the pharmaceutical composition of the present invention is formulated so as to bring about a serum concentration of 10 µg/mL or higher of the anti-FKN antibody. In an alternative preferred embodiment of the present invention, the pharmaceutical composition of the present invention is formulated so as to bring about a serum concentration of 20 µg/mL or higher of the anti-FKN antibody.

In an alternative aspect, the present invention relates to a pharmaceutical composition comprising an anti-FKN antibody, wherein the pharmaceutical composition is used so as to bring about a therapeutically effective serum anti-FKN antibody concentration after a single administration or multiple administrations to a human subject. The pharmaceutical composition of the present invention is not particularly limited and can be typically in the form of a formulation for injection prepared for subcutaneous administration. In one embodiment, the pharmaceutical composition of the present invention is used such that the mean trough concentration of the anti-FKN antibody is 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher. In a preferred embodiment, the pharmaceutical composition of the present invention is used such that the mean trough concentration of the anti-FKN antibody is 10 µg/mL or higher or 20 µg/mL or higher.

In the present invention, the pharmacokinetic characteristics of a concentration-time curve, for example, a maximum serum concentration (Cₘₐₓ), a time to reach Cₘₐₓ (tₘₐₓ), and an area under the serum concentration-time curve (AUC) are examined by statistical approaches sufficiently established in the field of pharmacokinetics. When the ratio of a population mean of an evaluation parameter as described above between a test formulation and a standard formulation is 0.80 to 1.25, these formulations are generally regarded as being biologically equivalent.

In the present invention, mean values of the pharmacokinetic parameters such as Cₘₐₓ and AUC can be calculated by any of geometric mean and arithmetic mean methods. In the present specification, mean Cₘₐₓ, mean AUC, mean tFKN concentration, and mean trough concentration are indicated by arithmetic mean values, and tₘₐₓ is indicated by a median value. Even if a mean value of interest is calculated by a method different from that described in the present specification, the mean value is intended to belong to the scope of claims according to the present invention as long as the corresponding mean value calculated according to the method of the present specification falls within the numerical range described in the scope of claims.

In the present invention, the term "one dose" or "per dose" regarding the dose of the anti-FKN antibody refers to the absolute amount of the anti-FKN antibody per administration. Thus, for example, the term "200 mg per dose" may mean that one set of the pharmaceutical composition of the present invention comprising 200 mg of the anti-FKN antibody is administered, or may mean that two sets of the pharmaceutical composition of the present invention comprising 100 mg of the anti-FKN antibody are administered at the same time.

In the present invention, for example, the term "dosing intervals of once a week to once every two weeks" regarding the dosing interval of the anti-FKN antibody means that the dosing interval from the n^{th} (n represents an integer) administration to the (n + 1)^{th} administration is 1 week to 2 weeks, and the dosing interval between the n^{th} administration and the (n + 1)^{th} administration may be different from the dosing interval between the (n + 1)^{th} administration and the (n + 2)^{th} administration. For example, the term "dosing intervals of once a week to once every two weeks" naturally includes the case where the dosing interval from the first administration to the second administration is 1 week and the dosing interval from the second administration to the third administration is 2 weeks.

In the present specification, the term "human" or "human subject" means a healthy adult male, and typically, any human who has rheumatoid arthritis or manifests a clinical sign and symptom of any disease or disorder that may cause rheumatoid arthritis. In the present invention, the term "human" or "human subject" is preferably a rheumatic disease patient who has not obtained adequate therapeutic effects from at least one type of antirheumatic drug or anti-TNF formulation, has not obtained sustained therapeutic effects therefrom, or has not respond thereto.

The term "rheumatoid arthritis" refers to a disease state that can be diagnosed according to the 1987 ACR Classification Criteria or the 2010 ACR/EULAR Classification Criteria. Examples of physiological indexes of rheumatoid arthritis include symmetrical joint swelling and pain on passive movement, which are characteristics, but not unchangeable, of rheumatoid arthritis. A candidate for the treatment described in the present invention can be resistant to methotrexate or an anti-TNF formulation (adalimumab, infliximab, golimumab, certolizumab pegol, or etanercept) from the viewpoint that methotrexate or the anti-TNF formulation is not effective or not completely effective for the treatment of its symptom.

### 2. Anti-FKN antibody

In the present invention, the term "anti-FKN antibody" refers to humanized anti-human fractalkine antibody H3-2L4 or an antibody functionally equivalent thereto. In the present invention, the "functionally equivalent antibody" refers to an antibody that is equivalent to the antibody H3-2L4 in terms of at least any of or preferably all of binding affinity, neutralizing activity, cross reactivity, and pharmacokinetics in blood against human FKN.

In the present invention, the term "anti-FKN antibody" may include an antigen-binding fragment thereof. Such an antigen-binding fragment is not particularly limited as long as the antigen-binding fragment is a functional and structural fragment of the anti-FKN antibody, maintains the binding activity of the antibody against FKN, and does not significantly differ in pharmacokinetics in blood from the complete antibody. Examples of the antigen-binding fragment of the antibody include, but are not limited to, Fab, Fab', F(ab')₂, Fv, single-chain (scFv), variants thereof, fusion proteins containing the antibody moiety, other modified structures of immunoglobulin molecules containing an antigen recognition site, and the like.

In one embodiment, the anti-FKN antibody of the present invention can be any antibody comprising the following CDR sequences:
(a) CDR-H1 comprising the amino acid sequence of SEQ ID NO: 15 (NYYIH);
(b) CDR-H2 comprising the amino acid sequence of SEQ ID NO: 16 (WIYPGDGSPKFNERFKG);
(c) CDR-H3 comprising the amino acid sequence of SEQ ID NO: 17 (GPTDGDYFDY);
(d) CDR-L1 comprising the amino acid sequence of SEQ ID NO: 18 (RASGNIHNFLA);
(e) CDR-L2 comprising the amino acid sequence of SEQ ID NO: 19 (NEKTLAD); and
(f) CDR-L3 comprising the amino acid sequence of SEQ ID NO: 20 (QQFWSTPYT).

In an alternative embodiment, the anti-FKN antibody can be an antibody comprising a heavy chain and a light chain, wherein a heavy chain variable region of the antibody comprises the amino acid sequence of SEQ ID NO: 21
(QVQLVQSGAEVKKPGASVKVSCKASGYTFTNYYIHWVKQAPGQGLEWIGWIYPGDG SPKFNERFKGRTTLTADKSTNTAYMLLSSLRSDDTAVYFCATGPTDGDYFDYWGQGT TVTVSS), SEQ ID NO:13
(QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS), SEQ ID NO: 22
(QVQLVQSGAEVKKPGASVKVSCKASGYTFTNYYIHWVRQAPGQGLEWIGWIYPGDG SPKFNERFKGRTTLTRDKSTNTAYMELSSLRSDDTAVYFCATGPTDGDYFDYWGQGT TVTVSS), SEQ ID NO: 23
(QVQLVQSGAEVKKPGASVKVSCKASGYTFTNYYIHWVRQAPGQGLEWIGWIYPGDG SPKFNERFKGRTTMTADTSTSTAYMELSSLRSEDTAVYFCARGPTDGDYFDYWGQGT TVTVSS), or SEQ ID NO: 24
(QVQLVQSGAEVKKPGASVKVSCKASGYTFTNYYIHWVRQAPGQGLEWIGWIYPGDG SPKFNERFKGRTTLTADKSTSTAYMELSSLRSEDTAVYFCARGPTDGDYFDYWGQGT TVTVSS), and a light chain variable region of the antibody comprises the amino acid sequence of SEQ ID NO: 25
(DIQMTQSPSSLSASVGDRVTITCRASGNIHNFLAWYQQKPGKAPKFLVYNEKTLAD GVPSRFSGSGSGTQYTLTISSLQPEDFATYFCQQFWSTPYTFGGGTKVEIK), SEQ ID NO: 14
(DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK), or SEQ ID NO: 26
(DIQMTQSPSSLSASVGDRVTITCRASGNIHNFLAWYQQKPGKAPKLLIYNEKTLAD GVPSRFSGSGSGTQYTLTISSLQPEDFATYFCQQFWSTPYTFGGGTKVEIK).

In a preferred embodiment, the anti-FKN antibody can be an antibody comprising a heavy chain and a light chain, wherein a heavy chain variable region of the antibody comprises the amino acid sequence of SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) and a light chain variable region of the antibody comprises the amino acid sequence of SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK).

In a particular embodiment, the anti-FKN antibody is an antibody comprising a constant region of human IgG2 isotype.

In a particular embodiment, the anti-FKN antibody is an antibody wherein a Fc region of the constant region of human IgG2 isotype contains V234A and/or G237A mutations.

In a particularly preferred embodiment of the present invention, the anti-FKN antibody is antibody H3-2L4 consisting of a heavy chain consisting of the amino acid sequence represented by SEQ ID NO: 11 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK) and a light chain consisting of the amino acid sequence represented by SEQ ID NO: 12 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIKRTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC).

Needless to say, an antibody derived from any of the anti-FKN antibodies listed above by appropriate alteration (e.g., modification of the antibody or partial substitution, addition, or deletion of the amino acid sequence of the antibody) so as to maintain the functions of the antibody or in order to add or improve the functions of the antibody is also included in the antibody of the present invention. More specifically, an antibody lacking lysine (Lys) positioned at the carboxy terminus (C terminus) of the heavy chain by an artificial method such as genetic engineering in order to decrease heterogeneity among antibodies produced by antibody-producing cells is also included in the scope of the present invention. In addition, the anti-FKN antibody contained in the pharmaceutical composition of the present invention is not necessarily required to have complete homogeneity. For example, an anti-FKN antibody lacking lysine (Lys) positioned at the carboxy terminus (C terminus) of the heavy chain and an anti-FKN antibody that does not lack this lysine may coexist in the pharmaceutical composition of the present invention as long as the pharmaceutical composition of the present invention maintains the intended functions.

The anti-FKN antibody may be modified, if desired. The modification of the anti-FKN antibody may be a modification that changes (a) the three-dimensional structure of an amino acid sequence in a modification region, such as sheet or helix conformation; (b) the electric charge or hydrophobic status of the molecule at a target site; or (c) the effects of a modification on the maintenance of side chain volume, or may be a modification by which these changes are not clearly observed.

The modification of the anti-FKN antibody may be achieved by, for example, the substitution, deletion, and/or addition of a constituent amino acid residue(s).

In the present specification, the amino acid is used in the broadest sense thereof and includes not only natural amino acids, for example, serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp), and proline (Pro) but non-natural amino acids such as amino acid variants and derivatives. Those skilled in the art should understand, by taking this wide definition into consideration, that examples of the amino acid in the present specification include: L-amino acids; D-amino acids; chemically modified amino acids such as amino acid variants and amino acid derivatives; amino acids, such as norleucine, β-alanine, and ornithine, which do not serve as materials constituting proteins *in vivo;* and chemically synthesized compounds having the characteristics of amino acids generally known to those skilled in the art. Examples of the non-natural amino acids include α-methylamino acids (α-methylalanine, etc.), D-amino acids (D-aspartic acid, D-glutamic acid, etc.), histidine-like amino acids (2-amino-histidine, β-hydroxy-histidine, homohistidine, α-fluoromethyl-histidine, α-methyl-histidine, etc.), amino acids having extra methylene in their side chains ("homo" amino acids), and amino acids in which a carboxylic acid functional group in the side chain is replaced with a sulfonic acid group (cysteic acid, etc.).

Naturally occurring amino acid residues can be classified into, for example, the following groups based on general side chain characteristics:
(1) hydrophobic residues: Met, Ala, Val, Leu, and Ile;
(2) neutral hydrophilic residues: Cys, Ser, and Thr;
(3) acidic residues: Asp and Glu;
(4) basic residues: Asn, Gln, His, Lys, and Arg;
(5) residues influencing chain orientation: Gly and Pro; and
(6) aromatic residues: Trp, Tyr, and Phe.

The non-conservative substitution of an amino acid sequence constituting the anti-FKN antibody may be performed by replacing an amino acid belonging to one of these groups with an amino acid belonging to any of the other groups. More conservative substitution may be performed by replacing an amino acid belonging to one of these groups with another amino acid belonging to the same group thereas. Likewise, the deletion or the substitution in an amino acid sequence may be appropriately performed.

### 3. Pharmaceutical composition and formulation

The pharmaceutical composition of the present invention comprises a therapeutically effective amount of the anti-FKN antibody. Such an amount of the anti-FKN antibody contained in the pharmaceutical composition of the present invention can vary according to the administration route and dosing interval of the pharmaceutical composition of the present invention.

In one embodiment of the present invention, the pharmaceutical composition of the present invention can comprise the anti-FKN antibody in an amount from 100 mg to 400 mg. In a particular embodiment of the present invention, the formulation of the present invention can comprise the anti-FKN antibody in an amount of at least 100 mg, at least 150 mg, at least 200 mg, at least 250 mg, at least 300 mg, at least 350 mg, or at least 400 mg. In a further alternative embodiment, the formulation of the present invention can comprise the anti-FKN antibody in an amount of 100 mg, 200 mg, or 400 mg.

The dosage form of the pharmaceutical composition of the present invention is not particularly limited and is typically a formulation for injection prepared for subcutaneous administration. The pharmaceutical composition of the present invention can be prepared, for example, as a formulation for injection of the anti-FKN antibody, together with a pharmaceutically acceptable excipient, in injectable water, physiological saline, or phosphate-buffered saline without limitations. Examples of the pharmaceutically acceptable excipient used in the present invention include, but are not limited to, a stabilizer, a surfactant, and a preservative.

The stabilizer used in the present invention is, for example, a carbohydrate, a saccharide, or a sugar (e.g., sucrose) accepted by an authority as an appropriate additive or excipient for pharmaceutical formulations. The concentration of the stabilizer is 15 to 250 mM, 150 to 250 mM, or 200 mM. The formulation may contain a secondary stabilizer.

Appropriate examples of the pharmaceutically acceptable surfactant used in the present invention include, but are not limited to, polyoxyethylene sorbitan fatty acid ester (Tween), polyethylene polypropylene glycol, polyoxyethylene stearate, polyoxyethylene alkyl ether (e.g., polyoxyethylene monolauryl ether), alkylphenyl polyoxyethylene ether (Triton-X), polyoxyethylene-polyoxypropylene copolymers (Poloxamer and Pluronic), and sodium dodecyl sulfate (SDS). The most appropriate polyoxyethylene sorbitan fatty acid esters are polysorbate 20 (Tween 20) and polysorbate 80 (Tween 80). The concentration of the surfactant is 0.01 to 0.1% (w/v), 0.01 to 0.08% (w/v), or 0.025 to 0.075% (w/v), for example, 0.05% (w/v).

Examples of the preservative used in the present invention includes, but are not limited to, paraben, benzyl alcohol, sodium benzoate, phenol, benzalkonium chloride, thimerosal, chlorobutanol, benzoic acid, sodium bisulfite, sodium propionate, and arbitrary combinations or mixtures thereof.

Examples of the buffer used in the present invention include, but are not limited to, histidine, citrate, phosphate, glycine, acetate, and arbitrary combinations or mixtures thereof.

The formulation of the present invention may contain a buffer or a pH adjuster for controlling pH. In one embodiment, the formulation of the present invention has a pH in the range of 4.0 to 9.0, in the range of 5.0 to 9.0, in the range of 5.0 to 8.0, in the range of 5.0 to 7.5, in the range of 5.5 to 7.0, or in the range of 5.5 to 6.5.

Those skilled in the art should understand that the formulation of the present invention can be isotonic to human blood, i.e., the formulation of the present invention has essentially the same osmotic pressure as that of human blood. Such an isotonic formulation generally has an osmotic pressure of 250 mOSm to 350 mOSm. The isotonicity can be measured by use of, for example, a vapor pressure or ice freezing-type osmometer. Examples of the tonicity agent used in the present invention include, but are not limited to, saccharides, salts, and amino acids.

In addition to the excipients mentioned above, typical excipients and processes for the production of the formulation for injection for subcutaneous administration are known in the art. See, for example, Introduction to Pharmaceutical Dosage Forms, 1985, Ansel, H.C., Lea and Febiger, Philadelphia, Pa.; Remington's Pharmaceutical Sciences, 1995, Mack Publ. Co., Easton, Pa. This literature is incorporated herein by reference in its entirety.

In one embodiment of the present invention, the pharmaceutical composition of the present invention can be any of
a pharmaceutical composition for subcutaneous administration formulated such that,
when 100 mg of the anti-fractalkine antibody is subcutaneously administered by a single administration to a human,
mean Cₘₐₓ is a value that falls within the numerical range of 80% to 125% of 10 µg/mL,
mean AUC₍₀₋₃₃₆ₕ₎ is a value that falls within the numerical range of 80% to 125% of 2.4 × 10³ µg·h/mL, or
mean AUC₍₀₋ₜ₎ is a value that falls within the numerical range of 80% to 125% of 3.0 × 10³ µg·h/mL;
a pharmaceutical composition for subcutaneous administration formulated such that
when 200 mg of the anti-fractalkine antibody is subcutaneously administered by a single administration to a human,
mean Cₘₐₓ is a value that falls within the numerical range of 80% to 125% of 27 µg/mL,
mean AUC₍₀₋₃₃₆ₕ₎ is a value that falls within the numerical range of 80% to 125% of 7.4 × 10³ µg·h/mL, or
mean AUC₍₀₋ₜ₎ is a value that falls within the numerical range of 80% to 125% of 1.2 × 10⁴ µg·h/mL; and
a pharmaceutical composition for subcutaneous administration formulated such that
when 400 mg of the anti-fractalkine antibody is subcutaneously administered by a single administration to a human,
mean Cₘₐₓ is a value that falls within the numerical range of 80% to 125% of 43 µg/mL,
mean AUC₍₀₋₃₃₆ₕ₎ is a value that falls within the numerical range of 80% to 125% of 1.2 × 10⁴ µg·h/mL, or
mean AUC₍₀₋ₜ₎ is a value that falls within the numerical range of 80% to 125% of 2.7 × 10⁴ µg·h/mL.

In one embodiment of the present invention, the pharmaceutical composition of the present invention can be any of
a pharmaceutical composition for subcutaneous administration formulated such that
when 100 mg of the anti-fractalkine antibody is subcutaneously administered by a single administration to a human,
mean Cₘₐₓ is a value that falls within the numerical range of 80% to 125% of 9.7 µg/mL,
mean AUC₍₀₋ₜ₎ is a value that falls within the numerical range of 80% to 125% of 2990 µg·h/mL, or
mean tₘₐₓ is a value that falls within the numerical range of 80% to 125% of 144 h;
a pharmaceutical composition for subcutaneous administration formulated such that
after a single subcutaneous administration of 200 mg of the anti-fractalkine antibody to a human, as to the anti-fractalkine antibody,
mean Cₘₐₓ is a value that falls within the numerical range of 80% to 125% of 27 µg/mL,
mean AUC₍₀₋ₜ₎ is a value that falls within the numerical range of 80% to 125% of 11900 µg·h/mL, or
mean tₘₐₓ is a value that falls within the numerical range of 80% to 125% of 156 h; and
a pharmaceutical composition for subcutaneous administration formulated such that
when 400 mg of the anti-fractalkine antibody is subcutaneously administered by a single administration to a human,
mean Cₘₐₓ is a value that falls within the numerical range of 80% to 125% of 43 µg/mL,
mean AUC₍₀₋ₜ₎ is a value that falls within the numerical range of 80% to 125% of 26600 µg·h/mL, or
mean tₘₐₓ is a value that falls within the numerical range of 80% to 125% of 156 h.

In a preferred embodiment of the present invention, the anti-fractalkine antibody is antibody H3-2L4 consisting of a heavy chain consisting of the amino acid sequence represented by SEQ ID NO: 11 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK) and a light chain consisting of the amino acid sequence represented by SEQ ID NO: 12 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIKRTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC).

### 4. Dose, dosing interval, and the number of doses

The pharmaceutical composition of the present invention is administered to a human subject at a dose that brings about therapeutically effective amelioration in rheumatoid arthritis. Thus, the pharmaceutical composition of the present invention is administered such that the serum concentration or the mean trough concentration of the anti-FKN antibody is 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher. Preferably, the pharmaceutical composition of the present invention is administered by multiple administrations such that the mean trough concentration of the anti-FKN antibody is 10 µg/mL or higher or 20 µg/mL or higher.

In one embodiment of the present invention, the pharmaceutical composition of the present invention is used such that at least 100 mg per dose of the anti-fractalkine antibody is subcutaneously administered to a human. In an alternative embodiment of the present invention, the pharmaceutical composition of the present invention is used such that 100 mg to 400 mg per dose of the anti-fractalkine antibody is subcutaneously administered to a human. In one embodiment of the present invention, the pharmaceutical composition of the present invention is used such that 100 mg, 200 mg, or 400 mg per dose of the anti-fractalkine antibody is subcutaneously administered to a human. In a preferred embodiment of the present invention, the pharmaceutical composition of the present invention is used such that 200 mg to 400 mg per dose of the anti-fractalkine antibody is subcutaneously administered to a human. In an alternative preferred embodiment of the present invention, the pharmaceutical composition of the present invention is used such that 200 mg or 400 mg per dose of the anti-fractalkine antibody is subcutaneously administered to a human.

The number of doses and dosing interval of the pharmaceutical composition of the present invention can vary according to the amount of the anti-FKN antibody administered per dose and an administration route, etc.

In one embodiment of the present invention, the pharmaceutical composition of the present invention is used such that at least 100 mg per dose of the anti-FKN antibody is subcutaneously administered by multiple administrations to a human subject in need thereof at dosing intervals of once a week to once every two months. In an alternative embodiment of the present invention, the pharmaceutical composition of the present invention is used such that at least 100 mg per dose of the anti-FKN antibody is subcutaneously administered by multiple administrations to a human subject in need thereof at dosing intervals of once a week to once a month. In an alternative embodiment of the present invention, the pharmaceutical composition of the present invention is used such that at least 100 mg per dose of the anti-FKN antibody is subcutaneously administered by multiple administrations to a human subject in need thereof at dosing intervals of once a week to once every two weeks. In a particular embodiment of the present invention, the pharmaceutical composition of the present invention is used such that at least 100 mg per dose of the anti-FKN antibody is subcutaneously administered at dosing intervals of every other week after two once-a-week administrations.

In one embodiment of the present invention, the pharmaceutical composition of the present invention is used such that 100 mg to 400 mg per dose of the anti-FKN antibody is subcutaneously administered by multiple administrations to a human subject in need thereof at dosing intervals of once a week to once every two months. In an alternative embodiment of the present invention, the pharmaceutical composition of the present invention is used such that 100 mg to 400 mg per dose of the anti-FKN antibody is subcutaneously administered by multiple administrations to a human subject in need thereof at dosing intervals of once a week to once a month. In an alternative embodiment of the present invention, the pharmaceutical composition of the present invention is used such that 100 mg to 400 mg per dose of the anti-FKN antibody is subcutaneously administered by multiple administrations to a human subject in need thereof at dosing intervals of once a week to once every two weeks. In a particular embodiment of the present invention, the pharmaceutical composition of the present invention is used such that 100 mg to 400 mg per dose of the anti-FKN antibody is subcutaneously administered at dosing intervals of every other week after two once-a-week administrations.

In an alternative embodiment of the present invention, the pharmaceutical composition of the present invention is used such that 100 mg, 200 mg, or 400 mg per dose of the anti-FKN antibody is subcutaneously administered by multiple administrations to a human subject in need thereof at dosing intervals of once a week to once every two months. In an alternative embodiment of the present invention, the pharmaceutical composition of the present invention is used such that 100 mg, 200 mg, or 400 mg per dose of the anti-FKN antibody is subcutaneously administered by multiple administrations to a human subject in need thereof at dosing intervals of once a week to once a month. In an alternative embodiment of the present invention, the pharmaceutical composition of the present invention is used such that 100 mg, 200 mg, or 400 mg per dose of the anti-FKN antibody is subcutaneously administered by multiple administrations to a human subject in need thereof at dosing intervals of once a week to once every two weeks. In a particular embodiment of the present invention, the pharmaceutical composition of the present invention is used such that 100 mg, 200 mg, or 400 mg per dose of the anti-FKN antibody is subcutaneously administered at dosing intervals of every other week after two once-a-week administrations.

In an alternative embodiment of the present invention, the pharmaceutical composition of the present invention is used such that 100 mg to 200 mg per dose of the anti-FKN antibody is subcutaneously administered by multiple administrations to a human subject in need thereof at dosing intervals of once a week to once every two months. In an alternative embodiment of the present invention, the pharmaceutical composition of the present invention is used such that 100 mg to 200 mg per dose of the anti-FKN antibody is subcutaneously administered by multiple administrations to a human subject in need thereof at dosing intervals of once a week to once a month. In an alternative embodiment of the present invention, the pharmaceutical composition of the present invention is used such that 100 mg to 200 mg per dose of the anti-FKN antibody is subcutaneously administered by multiple administrations to a human subject in need thereof at dosing intervals of once a week to once every two weeks. In a particular embodiment of the present invention, the pharmaceutical composition of the present invention is used such that 100 mg to 200 mg per dose of the anti-FKN antibody is subcutaneously administered at dosing intervals of every other week after two once-a-week administrations.

In a preferred embodiment of the present invention, the pharmaceutical composition of the present invention is used such that 200 mg to 400 mg per dose of the anti-FKN antibody is subcutaneously administered by multiple administrations to a human subject in need thereof at dosing intervals of once a week to once every two months. In an alternative preferred embodiment of the present invention, the pharmaceutical composition of the present invention is used such that 200 mg to 400 mg per dose of the anti-FKN antibody is subcutaneously administered by multiple administrations to a human subject in need thereof at dosing intervals of once a week to once a month. In an alternative preferred embodiment of the present invention, the pharmaceutical composition of the present invention is used such that 200 mg to 400 mg per dose of the anti-FKN antibody is subcutaneously administered by multiple administrations to a human subject in need thereof at dosing intervals of once a week to once every two weeks. In an alternative preferred embodiment of the present invention, the pharmaceutical composition of the present invention is used such that 200 mg to 400 mg per dose of the anti-FKN antibody is subcutaneously administered at dosing intervals of every other week after two once-a-week administrations.

The number of doses of the pharmaceutical composition of the present invention is not particularly limited as long as the pharmaceutical composition of the present invention brings about therapeutically effective amelioration in rheumatoid arthritis. The number of doses of the pharmaceutical composition of the present invention can vary according to the amount of the anti-FKN antibody administered per dose, an administration route, and dosing intervals.

In one embodiment of the present invention, the pharmaceutical composition of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of once a week to once every two months.

In an alternative embodiment of the present invention, the pharmaceutical composition of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of once a week to once a month.

In a further alternative embodiment of the present invention, the pharmaceutical composition of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of once a week to once every two weeks.

In a particular embodiment of the present invention, the pharmaceutical composition of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of every other week after two once-a-week administrations.

In a particular embodiment of the present invention, the pharmaceutical composition of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of once a week to once every two months such that the mean trough concentration of the anti-FKN antibody is 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher.

In a further alternative embodiment of the present invention, the pharmaceutical composition of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of once a week to once a month such that the mean trough concentration of the anti-FKN antibody is 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher.

In a further alternative embodiment of the present invention, the pharmaceutical composition or the anti-FKN antibody of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of once a week to once every two weeks such that the mean trough concentration of the anti-FKN antibody is 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher.

In a further alternative embodiment of the present invention, the pharmaceutical composition or the anti-FKN antibody of the present invention can be subcutaneously administered at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times or more at dosing intervals of every other week after two once-a-week administrations such that the mean trough concentration of the anti-FKN antibody is 5 µg/mL or higher, 10 µg/mL or higher, 15 µg/mL or higher, 20 µg/mL or higher, 25 µg/mL or higher, 30 µg/mL or higher, 35 µg/mL or higher, or 40 µg/mL or higher.

Hereinafter, particular aspects of the present invention will be illustrated without limitations.

In one aspect, the present invention relates to a pharmaceutical composition for the treatment of rheumatoid arthritis,
the pharmaceutical composition comprising an anti-fractalkine antibody and a pharmaceutically acceptable excipient, wherein
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations, and
the pharmaceutical composition is used such that at least 100 mg per dose of the anti-fractalkine antibody is subcutaneously administered to a human.

In an alternative aspect, the present invention relates to a pharmaceutical composition for the treatment of rheumatoid arthritis,
the pharmaceutical composition comprising an anti-fractalkine antibody and a pharmaceutically acceptable excipient, wherein
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) ;
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK) ;
the antibody comprises a constant region of human IgG2 isotype; and
a Fc region of the constant region of human IgG2 isotype contains V234A and G237A mutations, and
the pharmaceutical composition is used such that 100 mg to 400 mg per dose of the anti-fractalkine antibody is subcutaneously administered by multiple administrations to a human.
In an alternative aspect, the present invention relates to a pharmaceutical composition for the treatment of rheumatoid arthritis,
the pharmaceutical composition comprising an anti-fractalkine antibody and a pharmaceutically acceptable excipient, wherein
the anti-fractalkine antibody is an antibody wherein:
   a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) ;
   a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK) ;
   the antibody comprises a constant region of human IgG2 isotype; and
   a Fc region of the constant region of human IgG2 isotype contains V234A and G237A mutations, and
   the pharmaceutical composition is used such that 100 mg to 200 mg per dose of the anti-fractalkine antibody is subcutaneously administered by multiple administrations to a human.

In a further alternative aspect, the present invention relates to a pharmaceutical composition for the treatment of rheumatoid arthritis,
the pharmaceutical composition comprising an anti-fractalkine antibody and a pharmaceutically acceptable excipient, wherein
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) ;
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK) ;
the antibody comprises a constant region of human IgG2 isotype; and
a Fc region contains V234A and G237A mutations, and
the pharmaceutical composition is used such that 200 mg to 400 mg per dose of the anti-fractalkine antibody is subcutaneously administered by multiple administrations to a human.

In a further alternative aspect, the present invention relates to a pharmaceutical composition for the treatment of rheumatoid arthritis,
the pharmaceutical composition comprising an anti-fractalkine antibody and a pharmaceutically acceptable excipient, wherein
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) ;
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK) ;
the antibody comprises a constant region of human IgG2 isotype; and
a Fc region of the constant region of human IgG2 isotype contains V234A and G237A mutations, and
the pharmaceutical composition is used such that 100 mg, 200 mg, or 400 mg per dose of the anti-fractalkine antibody is subcutaneously administered by multiple administrations to a human.

The pharmaceutical composition of the present invention is subcutaneously administered by multiple administrations at dosing intervals of once a week to once every two weeks to a human so as to bring about a trough concentration of 10 µg/mL or higher of the anti-FKN antibody. In a preferred embodiment, the pharmaceutical composition of the present invention is subcutaneously administered by multiple administrations at dosing intervals of once a week to once every two weeks to a human so as to bring about a trough concentration of 15 µg/mL or higher of the anti-FKN antibody. In a more preferred embodiment, the pharmaceutical composition of the present invention is subcutaneously administered by multiple administrations at dosing intervals of once a week to once every two weeks to a human so as to bring about a trough concentration of 20 µg/mL or higher of the anti-FKN antibody.

In a preferred embodiment, the pharmaceutical composition of the present invention is a pharmaceutical composition for subcutaneous administration formulated such that when 100 mg of the anti-fractalkine antibody is subcutaneously administered by a single administration to a human, any one, two, or three of the following pharmacokinetic parameters are brought about:
mean Cₘₐₓ is a value that falls within the numerical range of 80% to 125% of 10 µg/mL;
mean AUC₍₀₋₃₃₆ₕ) is a value that falls within the numerical range of 80% to 125% of 2.4 × 10³ µg·h/mL; and
mean AUC₍₀₋ₜ₎ is a value that falls within the numerical range of 80% to 125% of 3.0 × 10³ µg·h/mL.

In an alternative preferred embodiment, the pharmaceutical composition of the present invention is a pharmaceutical composition for subcutaneous administration formulated such that when 200 mg of the anti-fractalkine antibody is subcutaneously administered by a single administration to a human, any one, two, or three of the following pharmacokinetic parameters are brought about:
mean Cₘₐₓ is a value that falls within the numerical range of 80% to 125% of 27 µg/mL;
mean AUC₍₀₋₃₃₆ₕ) is a value that falls within the numerical range of 80% to 125% of 7.4 × 10³ µg·h/mL; and
mean AUC₍₀₋ₜ₎ is a value that falls within the numerical range of 80% to 125% of 1.2 × 10⁴ µg·h/mL.

In a further alternative preferred embodiment, the pharmaceutical composition of the present invention is a pharmaceutical composition for subcutaneous administration formulated such that when 400 mg of the anti-fractalkine antibody is subcutaneously administered by a single administration to a human, any one, two, or three of the following pharmacokinetic parameters are brought about:
mean Cₘₐₓ is a value that falls within the numerical range of 80% to 125% of 43 µg/mL;
mean AUC₍₀₋₃₃₆ₕ₎ is a value that falls within the numerical range of 80% to 125% of 1.2 × 10⁴ µg·h/mL; and
mean AUC₍₀₋ₜ₎ is a value that falls within the numerical range of 80% to 125% of 2.7 × 10⁴ µg·h/mL.

In an alternative embodiment of the present invention, the pharmaceutical composition of the present invention is a pharmaceutical composition for subcutaneous administration formulated such that when 100 mg of the anti-fractalkine antibody is subcutaneously administered by a single administration to a human, any one, two, or three of the following pharmacokinetic parameters are brought about:
mean Cₘₐₓ is a value that falls within the numerical range of 80% to 125% of 9.7 µg/mL,
mean AUC₍₀₋ₜ₎ is a value that falls within the numerical range of 80% to 125% of 2990 µg·h/mL; and
mean tₘₐₓ is a value that falls within the numerical range of 80% to 125% of 144 h.

In an alternative embodiment of the present invention, the pharmaceutical composition of the present invention is a pharmaceutical composition for subcutaneous administration formulated such that when 200 mg of the anti-fractalkine antibody is subcutaneously administered by a single administration to a human, any one, two, or three of the following pharmacokinetic parameters are brought about:
mean Cₘₐₓ is a value that falls within the numerical range of 80% to 125% of 27 µg/mL;
mean AUC₍₀₋ₜ₎ is a value that falls within the numerical range of 80% to 125% of 11900 µg·h/mL; and
mean Tₘₐₓ is a value that falls within the numerical range of 80% to 125% of 156 h.

In an alternative embodiment of the present invention, the pharmaceutical composition of the present invention is a pharmaceutical composition for subcutaneous administration formulated such that when 400 mg of the anti-fractalkine antibody is subcutaneously administered by a single administration to a human, any one, two, or three of the following pharmacokinetic parameters are brought about:
mean Cₘₐₓ is a value that falls within the numerical range of 80% to 125% of 43 µg/mL;
mean AUC₍₀₋ₜ₎ is a value that falls within the numerical range of 80% to 125% of 26600 µg·h/mL; or
mean Tₘₐₓ is a value that falls within the numerical range of 80% to 125% of 156 h.

In a preferred embodiment, the anti-fractalkine antibody is antibody H3-2L4 consisting of a heavy chain consisting of the amino acid sequence represented by SEQ ID NO: 11 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK) and a light chain consisting of the amino acid sequence represented by SEQ ID NO: 12 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIKRTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC).

In a preferred embodiment, the pharmaceutical composition is a pharmaceutical composition comprising 100 mg or 200 mg of the anti-fractalkine antibody.

In a preferred embodiment, the pharmaceutical composition is a pharmaceutical composition that is used such that 100 mg, 200 mg, or 400 mg per dose of the anti-fractalkine antibody is subcutaneously administered.

In a preferred embodiment, the pharmaceutical composition is a pharmaceutical composition that is subcutaneously administered by multiple administrations at dosing intervals of once a week to once every two weeks to a human.

In a preferred embodiment, the pharmaceutical composition is a pharmaceutical composition that is subcutaneously administered such that the mean trough concentration of the anti-fractalkine antibody is 10 µg/mL or higher.

In a preferred embodiment, the pharmaceutical composition is a pharmaceutical composition that is subcutaneously administered such that the mean trough concentration of the anti-fractalkine antibody is 20 µg/mL or higher.

In an alternative aspect, the present invention relates to a therapeutic agent for rheumatoid arthritis, comprising an anti-fractalkine antibody, wherein
the therapeutic agent for rheumatoid arthritis is used such that 100 mg to 400 mg of the anti-fractalkine antibody is subcutaneously administered at dosing intervals of once a week to once every two weeks to a human, and
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) ;
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations.

In one embodiment, the present invention relates to a therapeutic agent for rheumatoid arthritis, comprising an anti-fractalkine antibody, wherein
the therapeutic agent for rheumatoid arthritis is used such that 100 mg to 200 mg of the anti-fractalkine antibody is subcutaneously administered at dosing intervals of once a week to once every two weeks to a human, and
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations.

In one embodiment, the present invention relates to a therapeutic agent for rheumatoid arthritis, comprising an anti-fractalkine antibody, wherein
the therapeutic agent for rheumatoid arthritis is used such that 200 mg to 400 mg of the anti-fractalkine antibody is subcutaneously administered at dosing intervals of once a week to once every two weeks to a human, and
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations.
In one embodiment, the present invention relates to a therapeutic agent for rheumatoid arthritis, comprising an anti-fractalkine antibody, wherein
the therapeutic agent for rheumatoid arthritis is used such that 100 mg, 200 mg, or 400 mg of the anti-fractalkine antibody is subcutaneously administered at dosing intervals of once a week to once every two weeks to a human, and
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations.

In a preferred embodiment, the anti-fractalkine antibody is antibody H3-2L4 consisting of a heavy chain consisting of the amino acid sequence represented by SEQ ID NO: 11 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK) and a light chain consisting of the amino acid sequence represented by SEQ ID NO: 12 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIKRTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC).

In one embodiment, the present invention relates to the pharmaceutical composition comprising the anti-fractalkine antibody, wherein the pharmaceutical composition is subcutaneously administered such that the mean serum total FKN concentration at the steady state is 60 µg/mL or higher, 70 µg/mL or higher, 80 µg/mL or higher, 90 µg/mL or higher, 100 µg/mL or higher, 110 µg/mL or higher, 120 µg/mL or higher, 130 µg/mL or higher, 140 µg/mL or higher, 150 µg/mL or higher, or 160 µg/mL or higher.

In one embodiment of the present invention, the pharmaceutical composition of the present invention is a pharmaceutical composition for subcutaneous administration, wherein when 100 mg of the anti-fractalkine antibody is administered by multiple administrations to a human, the mean serum total FKN concentration at the steady state is 70 µg/mL to 100 µg/mL.

In one embodiment of the present invention, the pharmaceutical composition of the present invention is a pharmaceutical composition for subcutaneous administration, wherein when the pharmaceutical composition is used such that 100 mg of the anti-fractalkine antibody is subcutaneously administered at dosing intervals of once a week to once every two weeks to a human, the mean serum total FKN concentration at the steady state is 70 µg/mL to 100 µg/mL.

In one embodiment of the present invention, the pharmaceutical composition of the present invention is a pharmaceutical composition for subcutaneous administration, wherein when 200 mg of the anti-fractalkine antibody is administered by multiple administrations to a human, the mean serum total FKN concentration at the steady state is 160 µg/mL to 190 µg/mL.

In one embodiment of the present invention, the pharmaceutical composition of the present invention is a pharmaceutical composition for subcutaneous administration, wherein when the pharmaceutical composition is used such that 200 mg of the anti-fractalkine antibody is subcutaneously administered at dosing intervals of once a week to once every two weeks to a human, the mean serum total FKN concentration at the steady state is 160 µg/mL to 190 µg/mL.

In one embodiment of the present invention, the human is a rheumatic disease patient who has not obtained adequate effects from at least one type of antirheumatic drug or anti-TNF formulation, has not obtained sustained effects therefrom, or has not respond thereto.

Those skilled in the art should understand that the present invention may be carried out by any one of or appropriate combination of two or more of all aspects described in the present specification unless a technical contradiction arises. Further, those skilled in the art should understood that the present invention can be preferably carried out by an appropriate combination of all preferred or advantageous aspects described in the present specification unless a technical contradiction arises.

Literatures cited in the present specification should be interpreted as being incorporated herein by reference in their entirety. Those skilled in the art can understand related contents disclosed in these literatures by reference as a part of the present specification without departing from the spirit and scope of the present invention according to the context of the present specification.

Literatures cited in the present specification are provided merely for the purpose of disclosing related techniques before the filing date of the present application. It should not be understood that the present inventors admit to having no right preceding such disclosure due to the prior inventions or any other reasons. All statements of these literatures are based on information which has been available by the present applicant, and there is no admission that the contents of these statements are accurate.

The terms in the present specification are used for illustrating particular embodiments and are not intended to limit the invention.

The term "comprise" or "include" used in the present specification means that described items (members, steps, factors, numbers, etc.) are present and the presence of the other items (members, steps, factors, numbers, etc.) is not excluded therefrom, unless the context evidently requires different interpretation. The term "consist of" encompasses aspects described by the terms "consist of" and/or "consist essentially of".

All terms (including technical terms and scientific terms) used herein have the same meanings as those understood in a broad sense by those skilled in the art to which the present invention belongs, unless otherwise defined. The terms used herein should be interpreted as having meanings consistent with meanings in the present specification and related technical fields and should not be interpreted in an idealized or excessively formal sense, unless otherwise defined.

Terms such as "first" or "second" are used for expressing various factors. However, these factors are understood to be not limited by these terms themselves. These terms are used merely for differentiating one factor from the other factors. For example, the first factor may be described as the second factor, and vice versa, without departing from the scope of the present invention.

In the present specification, it should be understood that numerical values used for indicating component contents, numerical ranges, etc., are modified with the term "approximately" unless otherwise specified. For example, "10 µg" is interpreted as meaning "approximately 10 µg" unless otherwise specified. Those skilled in the art can naturally understand the extent thereof rationally according to the technical common sense and the context of the present specification.

It should be understood that each aspect indicated in a singular form used in the present specification and claims may be in a plural form, and vice versa, unless the context evidently requires different interpretation and unless a technical contradiction arises.

Hereinafter, the present invention will be described in more detail with reference to Examples. However, the present invention can be embodied by various aspects and is not intended to be limited by Examples described herein. Those skilled in the art can implement the present invention by various changes or modifications, additions, deletions, substitutions, etc., without departing from the spirit or scope of the present invention.

Abbreviations used in Examples are idiomatic abbreviations well known to those skilled in the art. Some of the abbreviations are shown below:
AUC: area under the serum concentration-time curve AUC_{(0-inf)} : area under the serum concentration-time curve from zero time extrap-olated to infinite time AUC₍₀₋ₜ₎ : area under the concentration-time curve from zero time to time of last quantifiable concentration AUC₍₀₋₃₃₆ₕ₎ : area under the concentration-time curve from zero (predose) to fixed time-point 336 h (2 weeks) after the end of infusion
CDAI: clinical disease activity index
CL: total clearance
CL/F: apparent clearance
CRP: C-reactive protein
Cₘₐₓ: maximum serum concentration
DNCₘₐₓ: dose-normalized Cₘₐₓ
DNAUC: dose-normalized AUC
ELISA: enzyme-linked immunosorbent assay
FKN: fractalkine
IgG: immunoglobulin G
NONMEN: nonlinear mixed effect model
SAS: statistical analysis system
SDAI: simple disease activity index
t_{1/2}: serum elimination half-life
tₘₐₓ: time to reach peak serum concentration
V_{d}: volume of distribution
V_{z}/F: apparent volume of distribution at terminal phase

### Examples

### Example 1: Preparation of humanized anti-human fractalkine antibody

Administration tests to humans given below employed the humanized anti-human fractalkine antibody H3-2L4. The preparation of H3-2L4, including humanization, was carried out as described in WO2011/052799. H3-2L4 used in Example 2 or later was prepared by methods described below in 1-1 and 1-2.

### 1-1. Expression vector

A humanized anti-human fractalkine antibody (H3-2L4) expression vector was prepared as follows.

First, a signal sequence (SEQ ID NO: 3) was added to the N terminus of the amino acid sequence (SEQ ID NO: 1) of the heavy chain variable region (H3-2) of the humanized anti-human fractalkine antibody (H3-2L4), and the amino acid sequence (SEQ ID NO: 4) of a human IgG2 constant region containing two inserted mutations (V234A and G237A) was added to the C terminus thereof (SEQ ID NO: 5). Next, a signal sequence (SEQ ID NO: 6) was added to the N terminus of the amino acid sequence (SEQ ID NO: 2) of the light chain variable region (L4) of the humanized anti-human fractalkine antibody (H3-2L4), and the amino acid sequence (SEQ ID NO: 7) of a human Igκ constant region was added to the C terminus thereof (SEQ ID NO: 8). These amino acid sequences (SEQ ID NOs: 5 and 8) were converted to gene sequences optimal for expression in CHO cells. Respective sequences in which a recognition sequence for restriction enzyme HindIII and a Kozak sequence were added to the 5' ends of the gene sequences and a stop codon and a recognition sequence for restriction enzyme EcoRI were added to the 3' ends thereof were totally synthesized (SEQ ID NOs: 9 and 10).

The sequences defined by SEQ ID NOs: 1 to 10 are each as follows:
Amino acid sequence of heavy chain variable region (H3-2) (SEQ ID NO: 1) 
QVQLVQSGAEVKKPGASVKVSCKASGYTFTNYYIHWVKQAPGQGLEWIGWIYPGDGS

PKFNERFKGRTTLTADKSTNTAYMLLSSLRSEDTAVYFCATGPTDGDYFDYWGQGTT

VTVSS
Amino acid sequence of light chain variable region (L4) (SEQ ID NO: 2) 
DIQMTQSPSSLSASVGDRVTITCRASGNIHNFLAWYQQKPGKAPKLLIYNEKTLADG

VPSRFSGSGSGTDYTLTISSLQPEDFATYFCQQFWSTPYTFGGGTKVEIK
Signal sequence (SEQ ID NO: 3) MEWSWVFLFFLSVTTGVHS
Amino acid sequence of human IgG2 constant region containing two inserted mutations (V234A and G237A) (SEQ ID NO: 4) 
ASTKGPSVFPLAPCSRSTSESTAALGCLVKDYFPEPVTVSWNSGALTSGVHTFPAVL

QSSGLYSLSSVVTVPSSNFGTQTYTCNVDHKPSNTKVDKTVERKCCVECPPCPAPPA

AAPSVFLFPPKPKDTLMISRTPEVTCVVVDVSHEDPEVQFNWYVDGVEVHNAKTKPR

EEQFNSTFRVVSVLTVVHQDWLNGKEYKCKVSNKGLPAPIEKTISKTKGQPREPQVY

TLPPSREEMTKNQVSLTCLVKGFYPSDIAVEWESNGQPENNYKTTPPMLDSDGSFFL

YSKLTVDKSRWQQGNVFSCSVMHEALHNHYTQKSLSLSPGK
(SEQ ID NO: 5) 
MEWSWVFLFFLSVTTGVHSQVQLVQSGAEVKKPGASVKVSCKASGYTFTNYYIHWVK

QAPGQGLEWIGWIYPGDGSPKFNERFKGRTTLTADKSTNTAYMLLSSLRSEDTAVYF
 
CATGPTDGDYFDYWGQGTTVTVSSASTKGPSVFPLAPCSRSTSESTAALGCLVKDYF
PEPVTVSWNSGALTSGVHTFPAVLQSSGLYSLSSVVTVPSSNFGTQTYTCNVDHKPS
NTKVDKTVERKCCVECPPCPAPPAAAPSVFLFPPKPKDTLMISRTPEVTCVVVDVSH
EDPEVQFNWYVDGVEVHNAKTKPREEQFNSTFRVVSVLTVVHQDWLNGKEYKCKVSN
KGLPAPIEKTISKTKGQPREPQVYTLPPSREEMTKNQVSLTCLVKGFYPSDIAVEWE
SNGQPENNYKTTPPMLDSDGSFFLYSKLTVDKSRWQQGNVFSCSVMHEALHNHYTQK
SLSLSPGK
Signal sequence (SEQ ID NO: 6) MSVPTQVLGLLLLWLTDARC
Amino acid sequence of human Igκ constant region (SEQ ID NO: 7)
(SEQ ID NO: 8) 
MSVPTQVLGLLLLWLTDARCDIQMTQSPSSLSASVGDRVTITCRASGNIHNFLAWYQ

QKPGKAPKLLIYNEKTLADGVPSRFSGSGSGTDYTLTISSLQPEDFATYFCQQFWST

PYTFGGGTKVEIKRTVAAPSVFIFPPSDEQLKSGTASVVCLLNNFYPREAKVQWKVD

NALQSGNSQESVTEQDSKDSTYSLSSTLTLSKADYEKHKVYACEVTHQGLSSPVTKS

FNRGEC
(SEQ ID NO: 9) 
AAGCTTGCCGCCACCATGGAATGGTCCTGGGTGTTCCTGTTCTTCCTGTCCGTGACC
ACCGGCGTGCACTCCCAGGTGCAGCTGGTGCAGTCTGGCGCCGAAGTGAAGAAACCT
GGCGCCTCCGTGAAGGTGTCCTGCAAGGCCTCCGGCTACACCTTCACCAACTACTAC
 
ATCCACTGGGTGAAACAGGCCCCAGGACAGGGCCTGGAATGGATCGGCTGGATCTAC
CCCGGCGACGGCTCCCCCAAGTTCAACGAGCGGTTCAAGGGCCGGACCACCCTGACC
GCCGACAAGTCCACCAACACCGCCTACATGCTGCTGTCCTCCCTGCGGAGCGAGGAT
ACCGCCGTGTACTTCTGCGCCACCGGCCCTACCGACGGCGACTACTTCGACTACTGG
GGCCAGGGCACCACCGTGACCGTGTCCTCTGCCTCCACCAAGGGCCCCTCCGTGTTC
CCTCTGGCCCCTTGCTCCCGGTCCACCTCTGAGTCTACCGCCGCTCTGGGCTGCCTG
GTCAAAGACTACTTCCCCGAGCCTGTGACAGTGTCCTGGAACTCTGGCGCCCTGACC
TCTGGAGTGCATACCTTCCCTGCCGTGCTGCAGTCATCCGGCCTGTACTCCCTGTCC
TCCGTGGTGACAGTGCCCTCCTCCAACTTCGGCACCCAGACCTACACCTGTAACGTG
GACCACAAGCCCTCCAACACCAAGGTGGACAAGACCGTGGAACGGAAGTGCTGCGTG
GAATGCCCCCCCTGTCCTGCCCCTCCTGCCGCCGCTCCTTCCGTGTTTCTGTTCCCC
CCAAAGCCCAAGGACACCCTGATGATCTCCCGGACCCCCGAAGTGACCTGCGTGGTG
GTGGACGTGTCCCACGAGGACCCCGAGGTGCAGTTCAATTGGTACGTGGACGGCGTG
GAAGTGCACAACGCCAAGACCAAGCCCAGAGAGGAACAGTTCAACTCCACCTTCCGG
GTGGTGTCCGTGCTGACCGTGGTGCACCAGGACTGGCTGAACGGCAAAGAGTACAAG
TGCAAGGTCTCCAACAAGGGCCTGCCTGCCCCCATCGAAAAGACCATCAGCAAGACC
AAGGGCCAGCCCCGCGAGCCCCAGGTGTACACACTGCCCCCCAGCCGGGAAGAGATG
ACCAAGAACCAGGTGTCCCTGACCTGTCTGGTGAAAGGCTTCTACCCCTCCGATATC
GCCGTGGAATGGGAGTCCAACGGACAGCCCGAGAACAACTACAAGACCACCCCCCCC
ATGCTGGACTCCGACGGCTCATTCTTCCTGTACTCCAAGCTGACAGTGGACAAGTCC
CGGTGGCAGCAGGGCAACGTGTTCTCCTGCAGCGTGATGCACGAGGCCCTGCACAAC
CACTACACCCAGAAGTCCCTGAGCCTGAGCCCCGGCAAGTGATGAATTC
(SEQ ID NO: 10)

AAGCTTGCCGCCACCATGTCCGTGCCCACCCAGGTGCTGGGCCTGCTGCTGCTGTGG

CTGACCGACGCCAGATGCGACATCCAGATGACCCAGTCCCCCTCCAGCCTGTCCGCC

TCTGTGGGCGACAGAGTGACCATCACCTGTCGGGCCTCCGGCAACATCCACAACTTT

CTGGCCTGGTATCAGCAGAAGCCCGGCAAGGCCCCCAAGCTGCTGATCTACAACGAA

AAGACCCTGGCCGACGGCGTGCCCTCCAGATTCTCCGGCTCTGGCTCCGGCACCGAC

TACACCCTGACCATCTCCAGCCTGCAGCCCGAGGACTTCGCCACCTACTTTTGCCAG

CAGTTCTGGTCCACCCCCTACACCTTCGGCGGAGGCACCAAGGTGGAAATCAAGCGG

ACCGTGGCCGCTCCCTCCGTGTTCATCTTCCCACCCTCCGACGAGCAGCTGAAGTCC

GGCACCGCCTCCGTGGTGTGCCTGCTGAACAACTTCTACCCCCGCGAGGCCAAGGTG

CAGTGGAAGGTGGACAACGCCCTGCAGTCCGGCAACTCCCAGGAATCCGTCACCGAG

CAGGACTCCAAGGACAGCACCTACTCCCTGTCCTCCACCCTGACCCTGTCCAAGGCC

GACTACGAGAAGCACAAGGTGTACGCCTGCGAAGTGACCCACCAGGGCCTGTCCAGC

CCCGTGACCAAGTCCTTCAACCGGGGCGAGTGCTGATGAATTC

The totally synthesized gene sequence encoding the heavy chain was cleaved with restriction enzymes HindIII and EcoRI and inserted to the HindIII-EcoRI site of a pEE6.4 vector (Lonza Group Ltd.). The totally synthesized gene sequence encoding the light chain was cleaved with restriction enzymes HindIII and EcoRI and inserted to the HindIII-EcoRI site of a pEE12.4 vector (Lonza Group Ltd.). Each vector was cleaved with restriction enzymes NotI and PvuI. The heavy chain and light chain vector fragments were ligated to construct an expression vector.

### 1-2. Construction of cell line expressing antibody H3-2L4 and obtainment of antibody H3-2L4

CHOK1SV cells (Lonza Group Ltd.) acclimatized to a medium of CD-CHO/6 mM L-glutamine were transfected with the prepared expression vector by electroporation. The cells thus transfected were selected in a 37°C/10% CO₂ environment in a medium of CD-CHO/50 µM MSX to obtain cells expressing the antibody of interest. Then, the cells were cloned to prepare a cell bank. The prepared cell bank was revived and cultured, and the culture supernatant was purified by chromatography to obtain the antibody H3-2L4 of interest.

The antibody H3-2L4 thus obtained had a heavy chain and a light chain having the amino acid sequences represented by SEQ ID NOs: 11 and 12, respectively.
Full-length heavy chain of H3-2L4 (SEQ ID NO: 11) 
QVQLVQSGAEVKKPGASVKVSCKASGYTFTNYYIHWVKQAPGQGLEWIGWIYPGDGS

PKFNERFKGRTTLTADKSTNTAYMLLSSLRSEDTAVYFCATGPTDGDYFDYWGQGTT

VTVSSASTKGPSVFPLAPCSRSTSESTAALGCLVKDYFPEPVTVSWNSGALTSGVHT

FPAVLQSSGLYSLSSVVTVPSSNFGTQTYTCNVDHKPSNTKVDKTVERKCCVECPPC

PAPPAAAPSVFLFPPKPKDTLMISRTPEVTCVVVDVSHEDPEVQFNWYVDGVEVHNA

KTKPREEQFNSTFRVVSVLTVVHQDWLNGKEYKCKVSNKGLPAPIEKTISKTKGQPR

EPQVYTLPPSREEMTKNQVSLTCLVKGFYPSDIAVEWESNGQPENNYKTTPPMLDSD

GSFFLYSKLTVDKSRWQQGNVFSCSVMHEALHNHYTQKSLSLSPGK
 Full-length light chain of H3-2L4 (SEQ ID NO: 12)

DIQMTQSPSSLSASVGDRVTITCRASGNIHNFLAWYQQKPGKAPKLLIYNEKTLADG

VPSRFSGSGSGTDYTLTISSLQPEDFATYFCQQFWSTPYTFGGGTKVEIKRTVAAPS

VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

The amino acid sequences of the heavy chain variable region and the light chain variable region of the antibody H3-2L4 were each as follows:
Heavy chain variable region of H3-2L4 (SEQ ID NO: 13) 
QVQLVQSGAEVKKPGASVKVSCKASGYTFTNYYIHWVKQAPGQGLEWIGWIYPGDGS

PKFNERFKGRTTLTADKSTNTAYMLLSSLRSEDTAVYFCATGPTDGDYFDYWGQGTT

VTVSS
Light chain variable region of H3-2L4 (SEQ ID NO: 14)

DIQMTQSPSSLSASVGDRVTITCRASGNIHNFLAWYQQKPGKAPKLLIYNEKTLADG

VPSRFSGSGSGTDYTLTISSLQPEDFATYFCQQFWSTPYTFGGGTKVEIK

The amino acid sequences of CDR-H1 to CDR-H3 and CDR-L1 to CDR-L3 of the antibody H3-2L4 were as follows:
CDR-H1 of H3-2L4 (SEQ ID NO: 15)
   NYYIH
CDR-H2 of H3-2L4 (SEQ ID NO: 16)
   WIYPGDGSPKFNERFKG
CDR-H3 of H3-2L4 (SEQ ID NO: 17)
   GPTDGDYFDY
CDR-L1 of H3-2L4 (SEQ ID NO: 18)
   RASGNIHNFLA
CDR-L2 of H3-2L4 (SEQ ID NO: 19)
   NEKTLAD
CDR-L3 of H3-2L4 (SEQ ID NO: 20)
   QQFWSTPYT

### Example 2: Single-administration test

A clinical phase 1 trial targeting healthy adult males was conducted for evaluating safety, tolerability, and pharmacokinetics by a single subcutaneous administration of H3-2L4.

### 2-1. [Clinical trial design]

This clinical trial was a monocentric, randomized, double-blind, placebo-controlled single-ascending dose study targeting healthy Japanese adult males and having a primary object to evaluate safety and tolerability by a single subcutaneous administration of H3-2L4. In this clinical trial, 32 subjects were divided into 4 cohorts (50, 100, 200, and 400 mg groups). H3-2L4 was subcutaneously administered by a single administration to 6 out of the 8 subjects in each cohort, and a placebo was subcutaneously administered by a single administration to the remaining 2 subjects.

This clinical trial was composed of a screening period, an observation hospitalization period, an administration hospitalization period, and a follow-up period.

The screening test was carried out within 28 days to 2 days before administration of a study drug, and the test for the observation hospitalization period was carried out on the day before administration to confirm eligibility. The subjects confirmed to be eligible were randomly allocated to a H3-2L4 or placebo group on the basis of an allocation list prepared by a person in charge of allocation. The dosing interval of each of the subjects was set to 30 minutes or longer. This clinical trial was a single-administration test, and the administration to each subject was performed only once independently of mealtime.

The subjects were observed and investigated by hospitalization for 2 weeks after the administration and then observed and investigated as outpatients until 8 weeks after the completion of the administration.

Cohort progression was conducted in the judgment of a principal investigator, and after a lapse of 7 or more days after the completion of administration to the final subject in a previous cohort, the administration to a next cohort was started.

The types of the study drug used in this trial and the formulations of the study drugs were as described below.

**[Table 1]**

| Table 1 Types of study drugs | | |
|---|---|---|
| Type | Dosage form and content | Manufacturer |
| H3-2L4 | Aqueous solution containing 100 mg of H3-2L4 in one vial (1 mL) | Eisai Co., Ltd. |
| Placebo | Aqueous solution free from H3-2L4 in one vial (1 mL) | |

**[Table 2]**

| Table 2 Formulation | |
|---|---|
| Component | Concentration |
| H3-2L4 | 100 mg/mL |
| Phosphate Buffer (pH 5.8) | 25 mM |
| Sucrose | 200 mM |
| Glycine | 50 mM |
| Polysorbate 80 | 0.05% |

### 2-2. [Administration of study drug]

H3-2L4 or a placebo was subcutaneously administered to the brachial region or the abdomen according to Table 3 below. For the first cohort (50 mg group) and the second cohort (100 mg group), 0.5 mL and 1.0 mL, respectively, were subcutaneously administered to either of the right or left upper arm. For the third cohort (200 mg group), 1.0 mL (2.0 mL in total) was subcutaneously administered to each of the right and left upper arms. For the fourth cohort (400 mg group), 1.0 mL (4.0 mL in total) was subcutaneously administered to each of a total of 4 regions, the right and left upper arms and the right and left flanks.

**[Table 3]**

| Table 3 Method for administering study drug | | |
|---|---|---|
| Cohort | Study drug | Administration method |
| 1 | H3-2L4 50 mg or placebo | Subcutaneous administration of 0.5 mL to either of the right or left upper arm |
| 2 | H3-2L4 100 mg or placebo | Subcutaneous administration of 1.0 mL to either of the right or left upper arm |
| 3 | H3-2L4 200 mg or placebo | Subcutaneous administration of 1.0 mL (2.0 mL in total) to each of the right and upper arms |
| 4 | H3-2L4 400 mg or placebo | Subcutaneous administration of 1.0 mL (4.0 mL in total) to each of a total of 4 regions, the right and left upper arms and the right and left flanks |

### 2-3. [Analysis of serum H3-2L4 concentration]

The serum H3-2L4 concentration was measured by use of a validated measurement method. Specifically, serum H3-2L4 was bound to human fractalkine immobilized on a microplate and reacted with a ruthenium-labeled anti-H3-2L4 rabbit polyclonal antibody. Then, the electrochemical luminescence intensity was measured using Sector Imager 6000 (Meso Scale Discovery) for quantification. Blood collection for the serum H3-2L4 concentration measurement was carried out at the time of blood collection shown in Table 4 below.

**[Table 4]**

| Table 4 Time of blood collection for pharmacokinetic evaluation | | | | |
|---|---|---|---|---|
| Period | Time of blood collection (time elapsed from administration at day 1) | | | Tolerance of time of blood collection |
| Day 1 | Immediately before administration | | | -3 hr |
| | 6 hr | | | ±5 min |
| | 12 hr | | | |
| Day 2 | 24 hr | The same hour as that of administration at day 1 | | ±1 hr |
| Day 3 | 48 hr | | | |
| Day 4 | 72 hr | | | |
| Day 5 | 96 hr | | | |
| Day 6 | 120 hr | | | |
| Day 7 | 144 hr | | | |
| Day 8 | 168 hr | | | |
| Day 9 | 192 hr | | | |
| Day 11 | 240 hr | | | |
| Day 13 | 288 hr | | | |
| Day 15 | 336 hr | | | |
| Day 22 | 504 hr | 3 weeks later | On the same day of week as that of administration at day 1 | ±1 d |
| Day 29 | 672 hr | 4 weeks later | | |
| Day 36 | 840 hr | 5 weeks later | | |
| Day 43 | 1008 hr | 6 weeks later | | |
| Day 50 | 1176 hr | 7 weeks later | | |
| Day 57 | 1344 hr | 8 weeks later | | |

### 2-4. [Pharmacokinetic analysis]

The pharmacokinetic analysis was carried out on the basis of the serum H3-2L4 concentration data using a set of subjects having data that permitted calculation of one or more pharmacokinetic parameters (hereinafter, referred to as a pharmacokinetic analysis set). The summary statistic of the serum H3-2L4 concentration was calculated for each specified time with respect to each dose. A diagram showing change in the serum H3-2L4 concentration was prepared, and pharmacokinetic parameters including Cₘₐₓ, tₘₐₓ, AUC₍₀₋ₜ₎, AUC_{(0-inf)}, t_{1/2}, CL/F, and V_{z}/F were calculated by non-compartmental analysis using the serum H3-2L4 concentration. The analysis was carried out using SAS, WinNonlin, Pharsight Knowledgebase Server, Microsoft Excel, and S-PLUS.

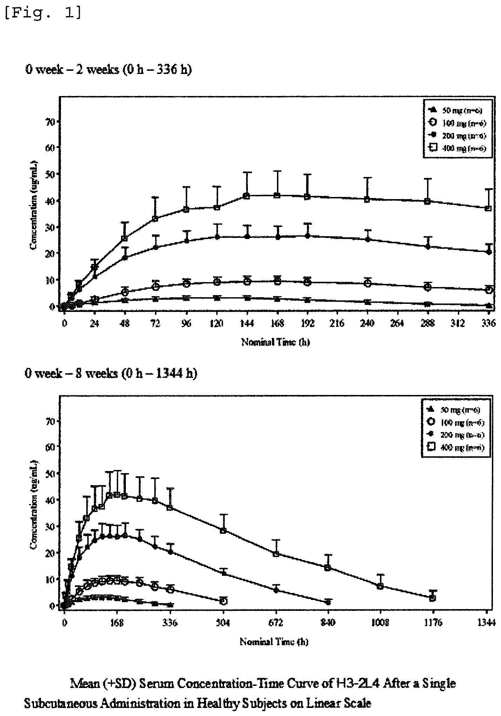

The results thus calculated are shown in Table 5 below and FIG. 1. In the table, each PK parameter other than tₘₐₓ is indicated by an arithmetic mean value, and the numerical value within the parentheses represents standard deviation. tₘₐₓ is indicated by a median value, and the numerical values within the parenthesis represent the smallest and largest values. In the table, a represents n = 3.

**[Table 5]**

| **Table 5 Summary of pharmacokinetic parameter when H3-2L4 was subcutaneously administered by single administration to healthy human adult** | | | | |
|---|---|---|---|---|
| **PK Parameter** | **H3-2L4** | | | |
| | **50mg (n=6)** | **100mg (n=6)** | **200mg (n=6)** | **400mg (n=6)** |
| Cₘₐₓ(µg/mL) | 3.14 (0.788) | 9.66 (1.88) | 27.3 (4.47) | 43.1 (8.88) |
| tₘₐₓ (h) | 120 (48 - 144) | 144 (120-240) | 156 (120-192) | 156 (72-288) |
| AUC₍₀₋ₜ₎ (µg•h/mL) | 602 (186) | 2990(745) | 11900(2170) | 26600(6770) |
| AUG₍₀₋₃₃₆ₕ₎ (µg•h/mL) | 662 (272) a | 2430(514) | 7420(1190) | 11800(2300) |
| t_{1/2,β} (h) | - | - | 242 (30.5) | 349 (37.2) |
| t_{1/2,γ} (h) | 40.8 (8.42) | 70.6 (19.5) | - | - |
| DN Cₘₐₓ ([µg/mL]/[mg]) | 0.0629 (0.0158) | 0.0966(0.0188) | 0.137 (0.0222) | 0.108 (0.0221) |
| DN AUC₍₀₋ₜ₎ ([µg•h/mL]/[mg]) | 12.0(3.72) | 29.9 (7.45) | 59.5 (10.8) | 66.6(16.9) |

As a result of the analysis, the pharmacokinetics when H3-2L4 was subcutaneously administered by a single administration was considered to exhibit a 3-phase profile having an absorption phase and two elimination phases (β phase and γ phase). After the single subcutaneous administration, H3-2L4 was slowly absorbed and reached Cₘₐₓ in 120 to 156 hours (median value). After the antibody reached Cₘₐₓ, a gradual elimination phase (β phase) was observed for the high concentrations, while a steeper final elimination phase (γ phase) was observed for the low concentrations. The boundary point between the γ phase and the β phase was considered to be around 10 µg/mL. The 50 and 100 mg groups exhibited a 2-phase profile, suggesting that the γ phase appeared subsequently to Cₘₐₓ. Since the 200 and 400 mg groups fell short of the number of blood samples necessary for accurately evaluating the γ phase as the final elimination phase, the pharmacokinetic parameters dependent on the γ phase were not able to be calculated. t_{1/2,β} was longer than t_{1/2,γ} and was prolonged with increase in dose. H3-2L4 exhibited nonlinear pharmacokinetics in the dose range of 50 to 400 mg.

### Example 3: Multiple-administration test

A clinical phase 1/2 trial targeting rheumatoid arthritis patients was conducted for evaluating safety, tolerability, pharmacokinetics, and effectiveness for rheumatoid arthritis by repeated subcutaneous administrations of H3-2L4.

### 3-1. [Clinical trial design]

This clinical trial was a multicenter, open-label, uncontrolled, multiple-ascending dose (MAD) study targeting Japanese patients with rheumatoid arthritis and having a primary object to evaluate safety and tolerability by repeated subcutaneous administrations of H3-2L4 for 12 weeks. Randomization was not carried out. In this clinical trial, a 100 mg administration group and a 200 mg administration group involved 12 subjects and 15 subjects, respectively, and after confirmation of the safety of the 100 mg administration group, the test progressed to the 200 mg administration group. The safety evaluation in an administration period of all of the subjects in the 200 mg administration group was further completed to confirm the absence of problems with safety. Then, the test was carried out targeting 10 subjects in a 400 mg administration group.

This clinical trial was composed of a screening period, an observation period, an administration period, a continuous administration period, and a follow-up period.

The screening test was carried out within 42 days to 2 days before administration of a study drug, and the test for the observation period was carried out on the day before initial administration of the study drug or before the administration on this day. H3-2L4 was administered to the subjects confirmed to be eligible.

Selection criteria for the eligibility were as follows:
(1) those at age 20 years or older and younger than 65 years;
(2) rheumatoid arthritis (RA) patients who satisfied the ACR Classification Criteria or the 2010 ACR/EULAR Classification Criteria;
(3) patients who received either of the following treatments, or both, before the start of screening and were found to have 4 or more tender joints (in 68 joints) and 4 or more swollen joints (in 66 joints) by evaluation in the screening period and the observation period:
   - patients who received 3-month or longer treatment with methotrexate (MTX). However, patients who had to discontinue the administration due to adverse reaction were not limited by the history of treatment for 3 months or longer, and
   - patients who received 3-month or longer treatment with an anti-TNF formulation. However, the anti-TNF formulation used in the treatment was limited to any one of adalimumab, infliximab, golimumab, certolizumab pegol, and etanercept (the anti-TNF formulation includes a biosimilar);
(4) patients who had not received any biological product (tocilizumab, abatacept, etc.) other than the anti-TNF formulation or two or more anti-TNF formulations in the past;
(5) patients who exhibited 0.6 mg/dL or higher of high sensitive CRP (hs-CRP) or an erythrocyte sedimentation rate (ESR) of 28 mm/hr or higher in the screening period; and
(6) patients who had a body weight of 30 kg or higher and 100 kg or lower at the time of screening.

In the administration period, H3-2L4 was administered a total of 7 times (week 0, week 1, week 2, and subsequently, every two weeks until week 10). The same dose was further administered 20 times every two weeks (40 weeks) to subjects who had no problem with safety in evaluation 2 weeks after the completion of the 7th administration (at week 12), exhibited 20% or more improvement in both of the tender joint count and the swollen joint count from the observation period, and desired continuous administration (continuous administration period). As for the continuous administration period of the 400 mg administration group, the dose was able to be decreased to 200 mg, which was also able to be brought back to 400 mg thereafter, in the judgment of a principal investigator or a subinvestigator.

The type of the study drug used in this trial and the formulation of the therapeutic agent were as follows.

**[Table 6]**

| Table 6 Study drug | | |
|---|---|---|
| Type | Dosage form and content | Manufacturer |
| H3-2L4 | Aqueous solution containing 100 mg of H3-2L4 in one vial (1 mL) | Eisai Co., Ltd. |

**[Table 7]**

| Table 7 Formulation | |
|---|---|
| Component | Concentration |
| H3-2L4 | 100 mg/mL |
| Phosphate Buffer (pH 5.8) | 25 mM |
| Sucrose | 200 mM |
| Glycine | 50 mM |
| Polysorbate 80 | 0.05% |

### 3-2. [Administration of study drug]

H3-2L4 was subcutaneously administered to the upper arms, the abdomen, or the femoral regions according to Table 8 below. For the 100 mg administration group, 1.0 mL was subcutaneously administered to any one of the right and left upper arms, the right and left flanks, and the right and left femoral regions. For the 200 mg administration group, 1.0 mL (2.0 mL in total) was subcutaneously administered to each of any two of the right and left upper arms, the right and left flanks, and the right and left femoral regions. For the 400 mg administration group, 1.0 mL (4.0 mL in total) was subcutaneously administered to each of any four of the right and left upper arms, the right and left flanks, and the right and left femoral regions. As for the 400 mg administration group, 2.0 mL (4.0 mL in total) was able to be subcutaneously administered to each of any two thereof as long as a principal investigator or a subinvestigator judged that this subcutaneous administration was properly achieved.

**[Table 8]**

| Table 8 Dose and administration method of H3-2L4 | | |
|---|---|---|
| Administration group | Study drug | Administration method |
| 100 mg administration group | H3-2L4 100 mg | Subcutaneous administration of 1.0 mL to any one of the right and left upper arms, the right and left flanks, and the right and left femoral regions |
| 200 mg administration group | H3-2L4 200 mg | Subcutaneous administration of 1.0 mL (2.0 mL in total) to each of any two of the right and left upper arms, the right and left flanks, and the right and left femoral regions |
| 400 mg administration group | H3-2L4 400 mg | Subcutaneous administration of 1.0 mL (4.0 mL in total) to each of any four of the right and left upper arms, the right and left flanks, and the right and left femoral regions |
| | | (subcutaneous administration of 2.0 mL (4.0 mL in total) to each of any two thereof was also possible as long as a principal investigator or a subinvestigator judged that this subcutaneous administration was properly achieved) |

The administration of the study drug was carried out after the completion of all investigations (except for findings about administration sites) scheduled on that day.

The same dose was further administered 20 times every two weeks (40 weeks) to subjects who had no problem with safety in evaluation 2 weeks after the completion of the 7th administration (at week 12), exhibited 20% or more improvement in both of the tender joint count and the swollen joint count from the observation period, and desired continuous administration. The subjects who progressed to the continuous administration period were observed and investigated until 52 weeks after the initial administration. The administration of the study drug was carried out after the completion of all investigations (except for findings about administration sites) scheduled on that day. The dosing intervals of the study drug were set to 7 days or longer (6-day or longer resting period). If a dosing interval was shorter than 7 days because of changing the day of observation and investigation, only the observation and investigation were carried out with a cessation of the study drug.

### 3-3. [Pharmacokinetics and drug potency]

The serum H3-2L4 concentration and the serum total FKN concentration were measured by use of validated measurement methods. The serum H3-2L4 concentration was measured by the method described in 2-4 of Example 2. The serum total FKN concentration was measured by sandwich ELISA using two types of anti-human fractalkine monoclonal antibodies 1F3 and 3A5-2 described in WO2011/052799. Specifically, serum total FKN was captured onto 1F3 immobilized on a plate, then sandwiched with horseradish peroxidase (HRP)-labeled 3A5-2, and further reacted with an HRP substrate 3,3',5,5'-tetramethylbenzidine (TMB). The luminescence value was measured using an auto-reader (Microplate reader Envision, PerkinElmer Co., Ltd.) to determine the serum total FKN concentration.

The pharmacokinetic analysis was carried out on the basis of the serum H3-2L4 concentration data using a set of subjects who received the study drug and had one or more evaluable serum EH3-2L4 concentration data points (hereinafter, referred to as a pharmacokinetic analysis set). The summary statistic of the serum H3-2L4 concentration was calculated for each specified time with respect to each dose. A diagram showing change in the serum H3-2L4 concentration was prepared.

### 3-3-1. [Serum H3-2L4 concentration]

Blood collection for the serum H3-2L4 concentration measurement was carried out at the times (counted, starting at the administration start day (day 1 (week 0)) (the same holds true for the description below); before administration (at day 1) and at days 8, 15 ± 1, 29 ± 3, 43 ± 3, 57 ± 3, 71 ± 3, and 85 ± 3) shown in the implementation schedule. As a rule, the blood collection for the serum H3-2L4 concentration measurement was carried out at any one point after 5 days (±3 days) from the administration at week 2, week 4, or week 6.

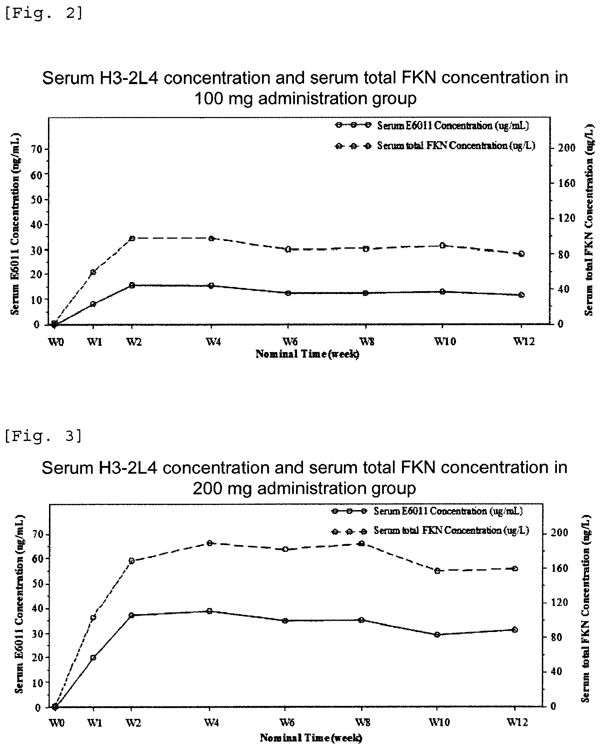

The serum H3-2L4 concentrations (indicated by arithmetic mean values) of the 100 mg administration group and the 200 mg administration group over the administration period (12 weeks) are shown in FIGs. 2 and 3, respectively.

### 3-3-2. [Serum total FKN concentration]

Blood collection for the serum total FKN concentration measurement was carried out at the times of blood collection (before administration (at day 1) and at days 8, 15 ± 1, 29 ± 3, 43 ± 3, 57 ± 3, 71 ± 3, and 85 ± 3) shown in the implementation schedule.

The summary statistics (indicated by arithmetic mean values) of the serum total FKN concentrations of the 100 mg administration group and the 200 mg administration group over the administration period (12 weeks) are shown in FIGs. 2 and 3, respectively.

### 3-4. [Effectiveness]

The effectiveness was analyzed using a set of subjects who received the study drug and had one or more evaluable post-administration effectiveness data points of the study drug (full analysis set (FAS)).

The effectiveness of the study drug for the rheumatoid arthritis was evaluated by calculating ACR20, ACR50, and ACR70 response rates and two-sided confidence intervals of 95% thereof for each time of evaluation with respect to each dose. The summary statistics of ACR evaluation items, i.e., tender and swollen joint counts, evaluation using VAS (patient assessment of pain, patient global assessment of disease activity, and provider global assessment of disease activity), patients' health assessment questionnaire (HAQ), and hs-CRP and ESR values and their amounts of change and rates of change were calculated for each time of evaluation. Likewise, the summary statistics of DAS28-ESR, DAS28-CRP, SDAI (simple disease activity index), and CDAI (clinical disease activity index) values and their amounts of change were calculated for each time of evaluation with respect to each dose. In addition, the respective remission rates of remission criteria (DAS28-ESR, DAS28-CRP, SDAI, CDAI, and Boolean) and two-sided confidence intervals of 95% thereof were calculated.

The analysis was carried out using SAS.

### 3-4-1. [ACR20, ACR50, and ACR70]

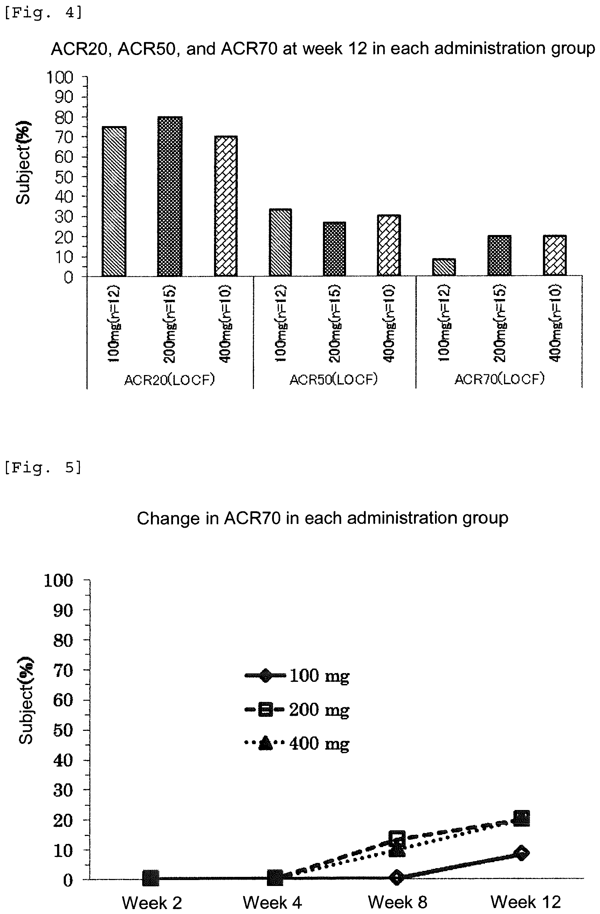

The results about ACR20, ACR50, and ACR70 (LOCF (last observation carried forward)) at week 12 in each administration group are shown in FIG. 4. ACR20, ACR50, and ACR70 at week 12 in the 100 mg administration group were 75.0%, 33.3%, and 8.3%, respectively. ACR20, ACR50, and ACR70 at week 12 in the 200 mg administration group were 80.0%, 26.7%, and 20.0%, respectively. ACR20, ACR50, and ACR70 at week 12 in the 400 mg administration group were 70.0%, 30.0%, and 20.0%, respectively.

Change in ACR70 (LOCF) until week 12 in each administration group is shown in FIG. 5. As shown in FIG. 5, the 200 mg administration group and the 400 mg administration group exhibited amelioration effects earlier than the 100 mg administration group.

### 3-4-2. [DAS28-CRP]

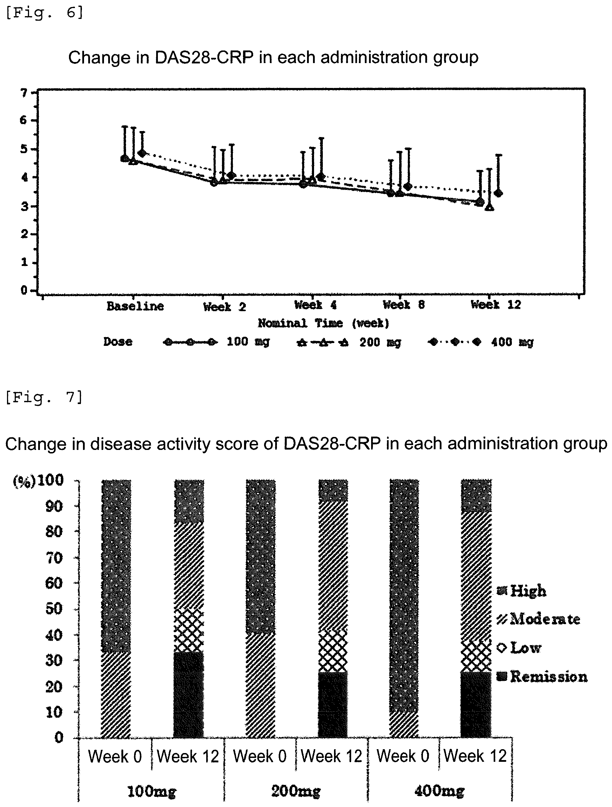

Change in DAS28-CRP (LOCF) in each administration group over the administration period (12 weeks) is shown in FIG. 6.

Change in the proportions of disease activity scores of DAS28-CRP in each administration group between before administration (at week 0) and at week 12 is shown in FIG. 7. As shown in FIG. 7, an improvement tendency was found in each administration group at 12 weeks after the start of administration. Remission was achieved in 20% or more of the subjects in each administration group.

### 3-4-3. [SDAI and Boolean]

The SDAI remission rates at week 12 in the 100 mg administration group and the 200 mg administration group were 16.7% and 20.0%, respectively.

The Boolean remission rates at week 12 in the 100 mg administration group and the 200 mg administration group were 8.3% and 26.7%, respectively.

### 3-4-4. [Erythrocyte sedimentation rate (ESR) and high sensitive CRP (hs-CRP)]

ESR was measured at the times (during the screening period, before administration (at day 1), and at days 15 ± 1, 29 ± 3, 57 ± 3, and 85 ± 3) shown in the implementation schedule, and at the time of discontinuation. ESR was further measured every 4 weeks until week 52 for the subjects who progressed to the continuous administration period. 1-hour ESR values were measured in the hospital, and the measurement values were described in case reports.

hs-CRP was measured at the times (during the screening period, before administration (at day 1), and at days 2, 9, 15 ± 1, 29 ± 3, 57 ± 3, and 85 ± 3) shown in the implementation schedule as a part of the biochemical examination of blood.

The serum H3-2L4 concentration at week 12, the area under the serum H3-2L4 concentration-time curve until week 12, and hs-CRP and ESR values at each time of evaluation (before administration (at week 0) and at week 12), and their rates of change were calculated for each subject in the 100 mg administration group and the 200 mg administration group. The results are shown in Tables 9 and 10 below. The values in the tables were not based on analysis using SAS.

**[Table 9]**

| Table 9 Results about hs-CRP and ESR in 100 mg administration group | | | | | | | | |
|---|---|---|---|---|---|---|---|---|
| Subject | Lowest serum H3-2L4 concentration at week 12 (µg/ml) | Area under lowest serum H3-2L4 concentration-time curve (µg·h/mL) | Before administration (week 0) | | Week 12 | | Rate of change at week 12 | |
| | | | hs-CRP (mg/dL) | ESR (mm/h) | hs-CRP (mg/dL) | ESR (mm/h) | Rate of change in CRP | Rate of change in ESR |
| A | | 58 | 2.94 | 74 | 3.26 | 64 | 0.11 | -0.14 |
| B | 19.9 | 1907 | 1.21 | 24 | 0.545 | 23 | -0.55 | -0.04 |
| C | 22.8 | 1657 | 0.355 | 41 | 0.508 | 33 | 0.43 | -0.20 |
| D | 8.89 | 1046 | 0.941 | 18 | 0.479 | 13 | -0.49 | -0.28 |
| E | 4.04 | 561 | 0.987 | 119 | 1.07 | 109 | 0.08 | -0.08 |
| F | 10.4 | 927 | 2.6 | 41 | 2.89 | 49 | 0.11 | 0.20 |
| G | 11 | 1072 | 0.317 | 50 | 0.143 | 26 | -0.55 | -0.48 |
| H | 5.56 | 632 | 0.197 | 38 | 0.324 | 40 | 0.64 | 0.05 |
| I | 10.6 | 1270 | 0.083 | 38 | 0.089 | 29 | 0.07 | -0.24 |
| J | 1.79 | 658 | 0.159 | 22 | 0.132 | 29 | -0.17 | 0.32 |
| K | 17.5 | 1379 | 0.268 | 67 | 0.075 | 22 | -0.72 | -0.67 |
| L | 16.6 | 1106 | 0.04 | 48 | 0.074 | 54 | 0.85 | 0.13 |

In the table, a rate of change in hs-CRP or a rate of change in ESR of -0.2 or less is indicated in bold type. A lowest serum H3-2L4 concentration at week 12 equal to or lower than the measurement limit is indicated in blank. [Table 10]

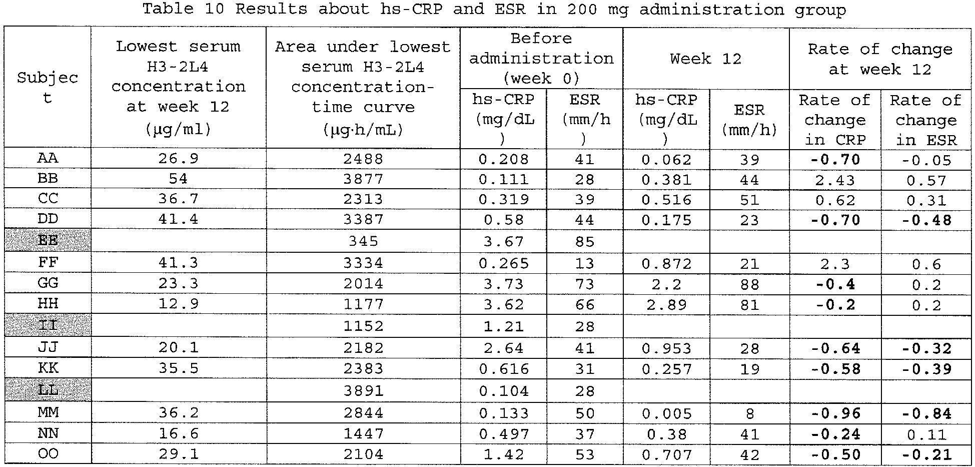

In the table, a rate of change in CRP or a rate of change in ESR of -0.2 or less is indicated in bold type. Test subjects EE, II, and LL refer to subjects who discontinued the test.

When the results described above were stratified to subjects having a hs-CRP value of 0.6 mg/dL or higher (selection criterion value for eligibility in this trial) before administration (at week 0), a serum H3-2L4 concentration-dependent improvement tendency in the rate of change in hs-CRP was found in subjects having a lowest serum H3-2L4 concentration of approximately 10 µg/mL or higher at week 12. In fact, the arithmetic mean lowest serum H3-2L4 concentration at week 12 was 7.2 µg/mL for a group of the subjects who had a hs-CRP value of 0.6 mg/dL or higher before administration (at week 0) and a measurable lowest serum H3-2L4 concentration at week 12 and did not exhibit 20% or more improvement in hs-CRP in the 100 mg administration group, whereas this arithmetic mean value was 14.4 µg/mL for a group of the subjects who exhibited 20% or more improvement in hs-CRP. Likewise, the arithmetic mean lowest serum H3-2L4 concentration at week 12 was 24.2 µg/mL for a group of the subjects who exhibited 20% or more improvement in hs-CRP in the 200 mg administration group. In light of these results and the fact that the values reached the steady state at and after week 2, it was considered necessary that a pharmaceutical composition comprising H3-2L4 is administered so as to bring about a mean trough concentration of at least 10 µg/mL.

### 3-4-5. [Evaluation of tender joint count and swollen joint count]

The tender joint count and the swollen joint count were evaluated during the screening period, during the observation period, at 2 weeks, 4 weeks, 8 weeks, and 12 weeks after the start of the study drug administration, and at the time of discontinuation. These counts were further evaluated every 4 weeks until week 52 for the subjects who progressed to the continuous administration period. As a rule, the evaluation was carried out by the same physician-in-charge in each medical institute.

The tender joint count was evaluated by targeting 68 joints and applying pressure to or moving the joints.

### 3-4-6. [Evaluation using visual analog scale (VAS)]

The evaluation using VAS was carried out during the observation period, at 2 weeks, 4 weeks, 8 weeks, and 12 weeks after the start of the study drug administration, and at the time of discontinuation. The evaluation was further conducted every 4 weeks until week 52 for the subjects who progressed to the continuous administration period. As a rule, the evaluation was carried out by the same physician-in-charge in each medical institute. 3-4-7. [Evaluation using health assessment questionnaire (HAQ)]

The evaluation using HAQ was carried out during the observation period, at 2 weeks, 4 weeks, 8 weeks, and 12 weeks after the start of the study drug administration, and at the time of discontinuation. The evaluation was further conducted every 4 weeks until week 52 for the subjects who progressed to the continuous administration period.

## Claims

1. A pharmaceutical composition for the treatment of rheumatoid arthritis,
the pharmaceutical composition comprising an anti-fractalkine antibody and a pharmaceutically acceptable excipient, wherein
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations, and
the pharmaceutical composition is used such that at least 100 mg per dose of the anti-fractalkine antibody is subcutaneously administered to a human.

2. The pharmaceutical composition according to claim 1, wherein
the pharmaceutical composition is used such that 100 mg to 400 mg per dose of the anti-fractalkine antibody is subcutaneously administered to a human.

3. The pharmaceutical composition according to claim 1, wherein
the pharmaceutical composition is used such that 100 mg, 200 mg, or 400 mg per dose of the anti-fractalkine antibody is subcutaneously administered to a human.

4. The pharmaceutical composition according to claim 1, wherein
when 100 mg of the anti-fractalkine antibody is subcutaneously administered by a single administration to a human,
mean Cₘₐₓ is a value that falls within the numerical range of 80% to 125% of 10 µg/mL,
mean AUC₍₀₋₃₃₆ₕ₎ is a value that falls within the numerical range of 80% to 125% of 2.4 × 10³ µg·h/mL, or
mean AUC₍₀₋ₜ₎ is a value that falls within the numerical range of 80% to 125% of 3.0 × 10³ µg·h/mL.

5. The pharmaceutical composition according to claim 1, wherein
when 200 mg of the anti-fractalkine antibody is subcutaneously administered by a single administration to a human,
mean Cₘₐₓ is a value that falls within the numerical range of 80% to 125% of 27 µg/mL,
mean AUC₍₀₋₃₃₆ₕ₎ is a value that falls within the numerical range of 80% to 125% of 7.4 × 10³ µg·h/mL, or
mean AUC₍₀₋ₜ₎ is a value that falls within the numerical range of 80% to 125% of 1.2 × 10⁴ µg·h/mL.

6. The pharmaceutical composition according to claim 1, wherein
when 400 mg of the anti-fractalkine antibody is subcutaneously administered by a single administration to a human,
mean Cₘₐₓ is a value that falls within the numerical range of 80% to 125% of 43 µg/mL,
mean AUC₍₀₋₃₃₆ₕ₎ is a value that falls within the numerical range of 80% to 125% of 1.2 × 10⁴ µg·h/mL, or
mean AUC₍₀₋ₜ₎ is a value that falls within the numerical range of 80% to 125% of 2.7 × 10⁴ µg·h/mL.

7. The pharmaceutical composition according to claim 1, wherein
the pharmaceutical composition is subcutaneously administered by multiple administrations at dosing intervals of once a week to once every two weeks.

8. The pharmaceutical composition according to any one of claims 1 to 7, wherein
the pharmaceutical composition is subcutaneously administered such that the mean trough concentration of the anti-fractalkine antibody is 10 µg/mL or higher.

9. The pharmaceutical composition according to any one of claims 1 to 7, wherein
the pharmaceutical composition is subcutaneously administered such that the mean trough concentration of the anti-fractalkine antibody is 20 µg/mL or higher.

10. A therapeutic agent for rheumatoid arthritis, comprising an anti-fractalkine antibody, wherein
the therapeutic agent for rheumatoid arthritis is used such that 100 mg to 400 mg of the anti-fractalkine antibody is subcutaneously administered at dosing intervals of once a week to once every two weeks to a human, and
the anti-fractalkine antibody is an antibody wherein:
a heavy chain variable region thereof comprises the amino acid sequence represented by SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS);
a light chain variable region thereof comprises the amino acid sequence represented by SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK);
the antibody comprises a constant region of human IgG2 isotype; and
a Fc region of the constant region of human IgG2 isotype contains V234A and G237A mutations.
